(19) Europäisches Patentamt / European Patent Office / Office européen des brevets

(11) **EP 1 283 891 B1**

(12) # EUROPEAN PATENT SPECIFICATION

(45) Date of publication and mention of the grant of the patent:
**13.06.2007 Bulletin 2007/24**

(21) Application number: **01927272.3**

(22) Date of filing: **20.04.2001**

(51) Int Cl.:
*C12N 15/82* (2006.01) *C12N 9/00* (2006.01)
*A01H 5/00* (2006.01)

(86) International application number:
**PCT/US2001/012928**

(87) International publication number:
**WO 2001/081604 (01.11.2001 Gazette 2001/44)**

(54) **PLANTS CONTAINING A CYTOSOLIC ACETYL COA-CARBOXYLASE NUCLEIC ACID**

PFLANZEN, WELCHE EINE CYTOSOLISCHE NUKLEINSäURE FüR ACETYL COA-CARBOXYLASE ENTHALTEN

PLANTES CONTENANT UN ACIDE NUCLEIQUE D'ACETYL COA-CARBOXYLASE CYTOSOLIQUE

(84) Designated Contracting States:
**DE FR GB NL**

(30) Priority: **20.04.2000 US 198794 P**

(43) Date of publication of application:
**19.02.2003 Bulletin 2003/08**

(73) Proprietor: **Cargill, Incorporated**
**Wayzata,**
**Minnesota 55391 (US)**

(72) Inventors:
- **SHORROSH, Basil, S.**
  **Ft. Collins, CO 80525 (US)**
- **DEBONTE, Lorin, R.**
  **Ft. Collins, CO 80525 (US)**

(74) Representative: **Fisher, Adrian John et al**
**CARPMAELS & RANSFORD**
**43-45 Bloomsbury Square**
**London WC1A 2RA (GB)**

(56) References cited:
**WO-A-95/29246** **WO-A-96/32484**
**US-A- 5 498 544** **US-A- 5 925 805**
**US-A- 5 962 767**

- **PATENT ABSTRACTS OF JAPAN vol. 1995, no. 09, 31 October 1995 (1995-10-31) -& JP 07 143887 A (MITSUI GYOSAI SHOKUBUTSU BIO KENKYUSHO:KK), 6 June 1995 (1995-06-06)**
- **PODKOWINSKI J ET AL: "STRUCTURE OF A GENE ENCODING A CYTOSOLIC ACETYL-COA CARBOXYLASE OF HEXAPLOID WHEAT" PROCEEDINGS OF THE NATIONAL ACADEMY OF SCIENCES OF USA, NATIONAL ACADEMY OF SCIENCE. WASHINGTON, US, vol. 93, no. 5, 1 March 1996 (1996-03-01), pages 1870-1874, XP002026103 ISSN: 0027-8424**
- **ROESLER K ET AL: "TARGETING OF THE ARABIDOPSIS HOMOMERIC ACETYL-COENZYME A CARBOXYLASE TO PLASTIDS OF RAPESEEDS" PLANT PHYSIOLOGY, AMERICAN SOCIETY OF PLANT PHYSIOLOGISTS, ROCKVILLE, MD, US, vol. 113, no. 1, 1997, pages 75-81, XP000891347 ISSN: 0032-0889**
- **YANAI YUKIHIRO ET AL: "Genomic organization of 251 kDa acetyl-CoA carboxylase genes in Arabidopsis: Tandem gene duplication has made two differentially expressed isozymes" PLANT AND CELL PHYSIOLOGY, vol. 36, no. 5, 1995, pages 779-787, XP008039089 ISSN: 0032-0781**
- **SASAKI Y ET AL: "THE COMPARTMENTATION OF ACETYL-COENZYME A CARBOXYLASE IN PLANTS" PLANT PHYSIOLOGY, AMERICAN SOCIETY OF PLANT PHYSIOLOGISTS, ROCKVILLE, MD, US, vol. 108, January 1995 (1995-01), pages 445-449, XP000619220 ISSN: 0032-0889**
- **SHORROSH ET AL.: 'Molecular cloning, characterization and elicitation of acetyl-CoA carboxylase from alfalfa' PROC. NATL. ACAD. SCI. USA vol. 91, May 1994, pages 4323 - 4327, XP002943400**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 283 891 B1

## Description

### TECHNICAL FIELD

This invention relates to oilseed plants, and more particularly to plants containing a nucleic acid encoding a cytosolic acetyl coA-carboxylase (ACCase) enzyme.

### BACKGROUND

Acetyl-CoA carboxylase [ACCase; EC 6.4.1.2] catalyzes the first committed step in fatty acid biosynthesis by converting acetyl-CoA to malonyl-CoA. In plants, a multisubunit (MS) form and a multifunctional (MF) form of ACCase have been identified. The MS form is composed of dissociable subunits of different sizes, including a biotin carboxyl carrier protein (BCCP), α- and β-carboxyltransferases (α-CT and β-CT, respectively), and a biotin carboxylase (BC). The MS form is present in plastids of dicotyledenous and of non-*Gramineae* monocotyledenous plants and is primarily involved in the biosynthesis of fatty acids.

The MF form of a plant ACCase is similar to mammalian ACCase (and is sometimes designated "eukaryotic" or "cytosolic" ACCase), in that it is a MF polypeptide with a molecular weight of more than 200 kDa. The MF form of ACCase from plants contains BCCP, BC, α-CT and β-CT functional domains in a single polypeptide. MF ACCase is most likely present in the cytosol of all plant species and in the chloroplasts of *Gramineae* plants. Plant MF ACCase is involved in the biosynthesis of very long chain fatty acids, flavonoids, and in the malonation of amino acids and aminocyclopropane-1-carboxylate (a precursor to ethylene).

Antisense nucleic acids against an MF ACCase have been introduced into *Brassica napus* (White et al., 1998, in Adv. in Plant Lipid Res., pp. 62-66, eds., Sánchez, J., Cerdá-Olmedo, E. & Martinez-Horce, E., Universidad De Sevilla, Spain) and an *Arabidopsis* genomic DNA encoding an MF ACCase under the control of a napin seed-specific promoter and linked to a small subunit (ss) Rubisco transit peptide was introduced into *B. napus* (Roesler et al., 1997, Plant Physiol., 113:75-81; US Patent No. 5,925,805).

### SUMMARY

Plants have been engineered to express a nucleic acid encoding an MF acetyl coA-carboxylase (ACCase), hereinafter referred to as cytosolic ACCase. Oil content was significantly increased in plants containing the cytosolic ACCase coding sequences.

In general, the invention feature plants containing a nucleic acid construct carrying a nucleic acid encoding a cytosolic ACCase operably linked to a promoter and lacking a transit peptide. This plant produces seeds that exhibit a statistically significant increase in oil content as compared to seeds produced by a corresponding plant lacking such a construct.

The invention additionally features plants containing a nucleic acid construct carrying a nucleic acid encoding a cytosolic ACCase lacking introns operably linked to a promoter. This plant produces seeds that exhibit a statistically significant increase in oil content as compared to seeds produced by a corresponding plant lacking such a construct.

The invention also features methods of producing a transgenic plant. This method includes selecting progeny transgenic plants of a plant containing a nucleic acid construct carrying a nucleic acid encoding a cytosolic ACCase operably linked to a promoter. Following at least one generation of selection, one or more of the progeny transgenic plants produce seeds exhibiting a statistically significant increase in oil content as compared to seeds produced by a corresponding plant lacking such a construct.

The invention further features methods of producing a plant by introducing a construct carrying a nucleic acid encoding a cytosolic ACCase operably linked to a promoter into one or more plants. Progeny of these plants, following at least one generation of selection, produce seeds that exhibit a statistically significant increase in oil content when compared to seeds produced by a corresponding plant lacking such a construct.

Yet another feature of the invention are methods of increasing the oil content in seeds by creating a plant containing a nucleic acid construct carrying a gene encoding a cytosolic ACCase operably linked to a promoter; and selecting progeny of the plant that exhibit a statistically significant increase in oil content in seeds as compared to seeds produced by a corresponding plant lacking such a construct.

Additionally featured in the invention are seeds produced by the above-described plants, and progeny of those plants, wherein the progeny produce seeds that exhibit a statistically significant increase in oil content when compared to seeds produced by the progeny of plants lacking such a construct.

Typically, the increase in oil content is from about 5% to about 25% on a dry weight basis. The above-described selection steps can include selecting progeny that contain the nucleic acid construct. Generally, soybean plants or *Brassica* plants, for example, *Brassica napus, B. rapa, B. juncea, B. carinata, B. nigra* and *B. oleracea* are useful in the invention.

Still yet another feature of the invention is a nucleic acid construct carrying a cytosolic ACCase coding sequence operably linked to a promoter but lacking a transit peptide and a nucleic acid construct carrying a cytosolic ACCase coding sequence lacking introns operably linked to a promoter.

A promoter included in a construct of the invention can be a cauliflower mosaic virus (CaMV) 35S promoter. The ACCase constructs described herein may not include nucleic acid sequences encoding a transit peptide operably linked to the nucleic acid sequences encoding the cytosolic ACCase. An example of a transit peptide is a tobacco small subunit Rubisco transit peptide. In addition, a nucleic acid encoding a cytosolic ACCase can encode a plant cytosolic ACCase, for example, an alfalfa cytosolic ACCase. Further and unless otherwise indicated, a nucleic acid encoding the ACCase can lack introns.

Unless otherwise defined, all technical and scientific terms used herein have the same meaning as commonly understood by one of ordinary skill in the art to which this invention pertains. The materials methods, and examples are illustrative only and not intended to be limiting. Suitable methods and materials are described below, although methods and materials similar or equivalent to those described herein can be used in the practice or testing of the present invention. In case of conflict, the present specification, including definitions, will control.

The details of one or more embodiments of the invention are set forth in the accompanying drawings and the description below. Other features, objects, and advantages of the invention will be apparent from the drawings and detailed description, and from the claims.

## DESCRIPTION OF THE DRAWINGS

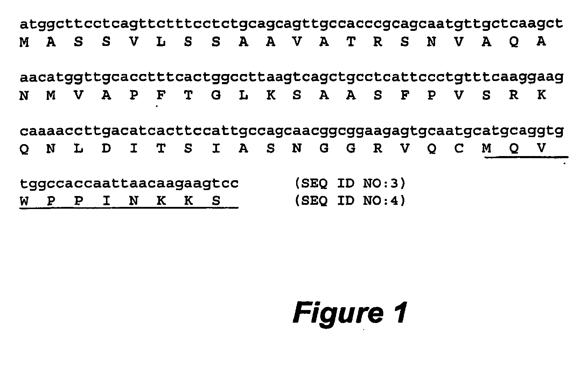

*Figure 1* is the nucleotide and amino acid sequence (SEQ ID NO:3 and 4 respectively) of the tobacco small subunit (ss) Rubisco transit peptide and 5' portion of the mature ss Rubisco protein (underlined).

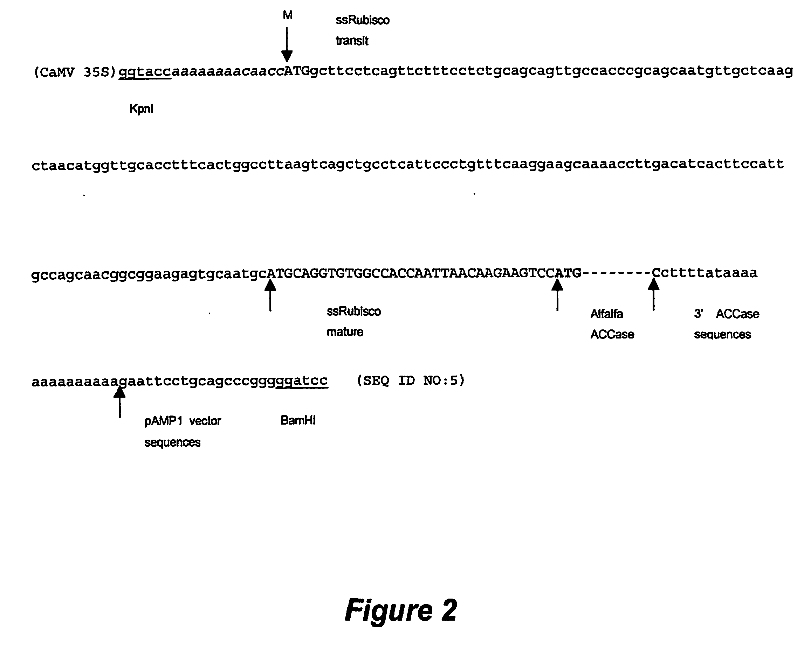

*Figure 2* is a representative +6ACCase construct (SEQ ID NO: 5). The nucleotide sequence encoding a transit peptide and the 5' portion of a small subunit (ss) Rubisco gene from tobacco is shown operably linked to an alfalfa cytosolic ACCase coding sequence. A consensus sequence for initiation of translation is italicized and includes the 3' end of a 35S cauliflower mosaic virus (CaMV) promoter and the 5' sequence encoding the tobacco ssRubisco transit peptide. The ACCase sequence corresponds to a portion of the coding sequence and 3' untranslated sequences (See Genbank Accession No. L25042); for the entire ACCase coding sequence). Arrows indicate the methionine-initiated (M) start codon of the ssRubisco transit peptide, the beginning of the portion of the ssRubisco mature protein included in the construct, the beginning and end of the ACCase coding sequence as published in GenBank, and the end of the ACCase 3' untranslated sequences. The *BamHI* and *KpnI* restriction sites were used to clone the +6ACCase construct into the ptet vector.

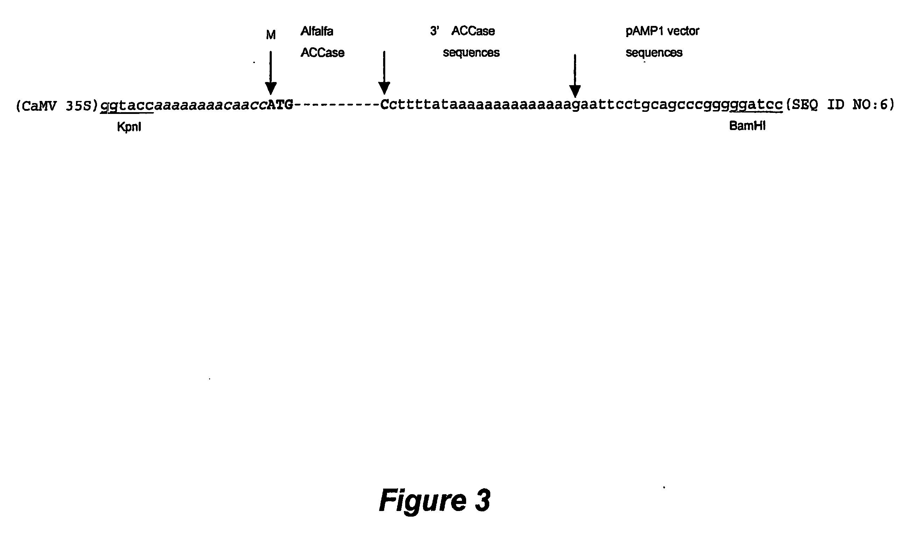

*Figure 3* is a representative -7ACCase construct (SEQ ID NO:6). The italicized consensus sequence for the initiation of translation includes the 3' end of a 35S cauliflower mosaic virus (CaMV) promoter and the 5' portion of an alfalfa cytosolic acetyl coA-carboxylase (ACCase) coding sequence (Shorrosh et al., 1994). The ACCase sequences are as described in the legend to Figure 2. Arrows indicate the methionine-initiated (M) start codon, the end of the ACCase coding as published in GenBank and the end of the ACCase 3' untranslated sequences. The *Bam*HI and *Kpn*I restriction sites were used to clone the -7ACCase construct into the ptet vector.

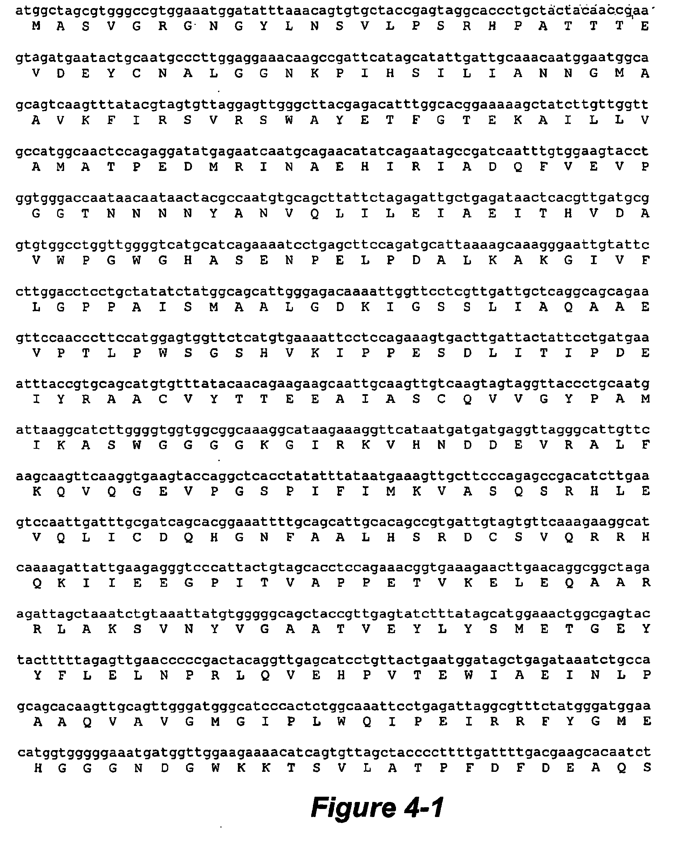

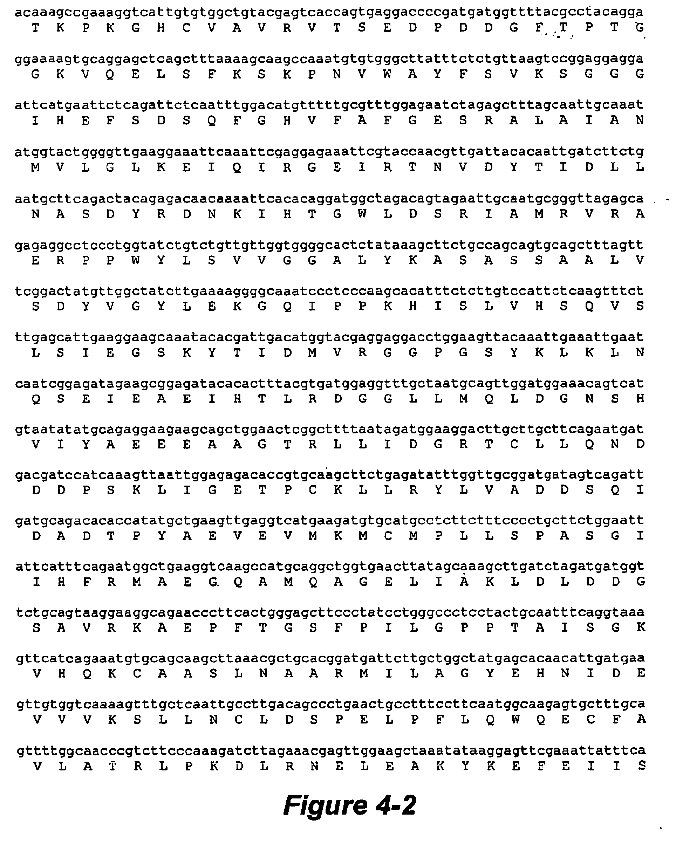

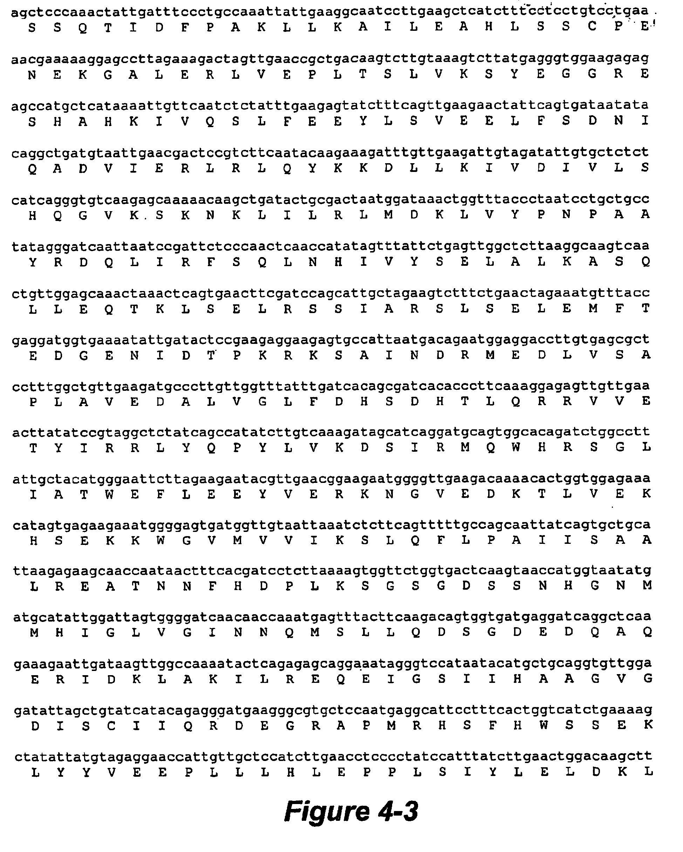

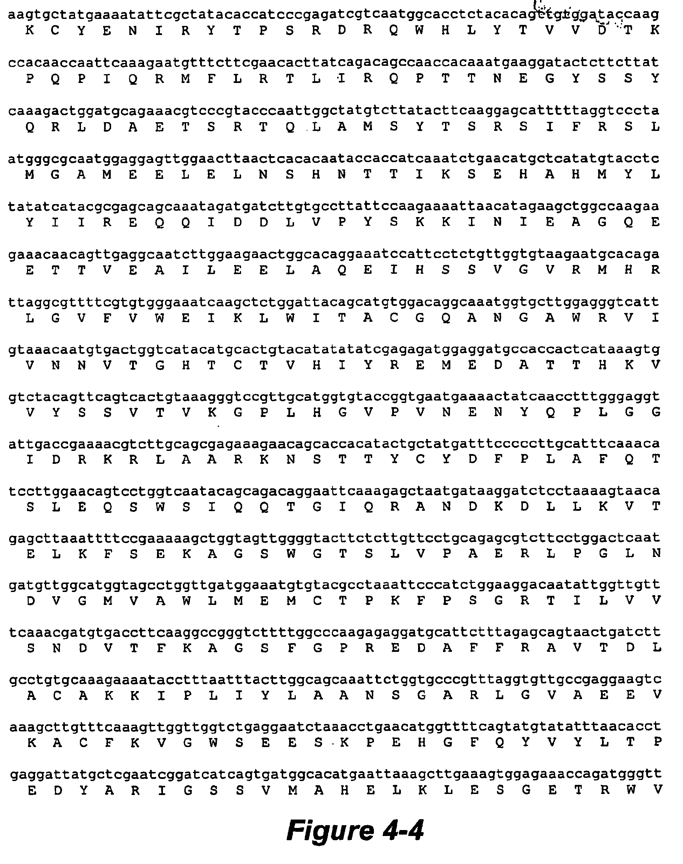

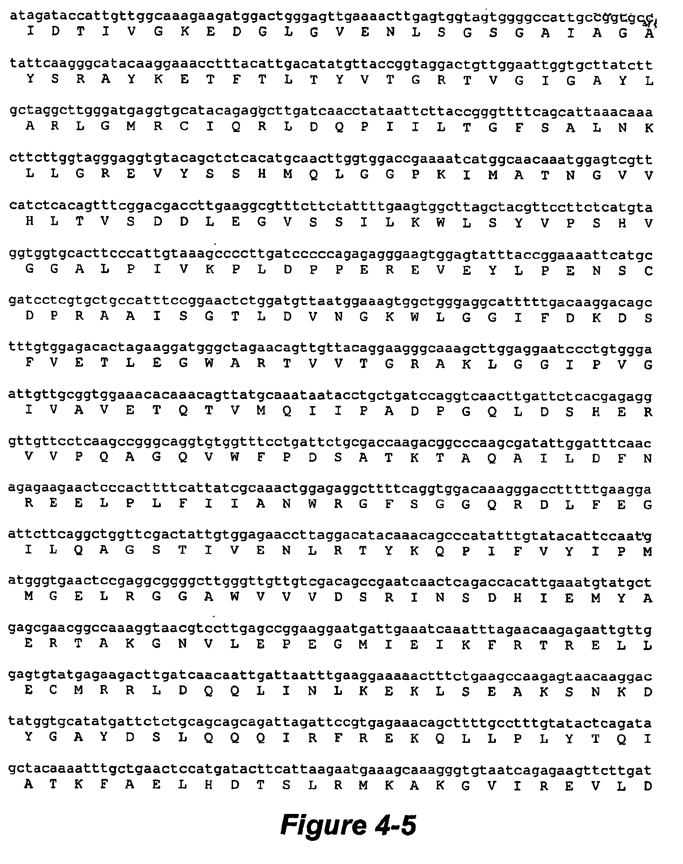

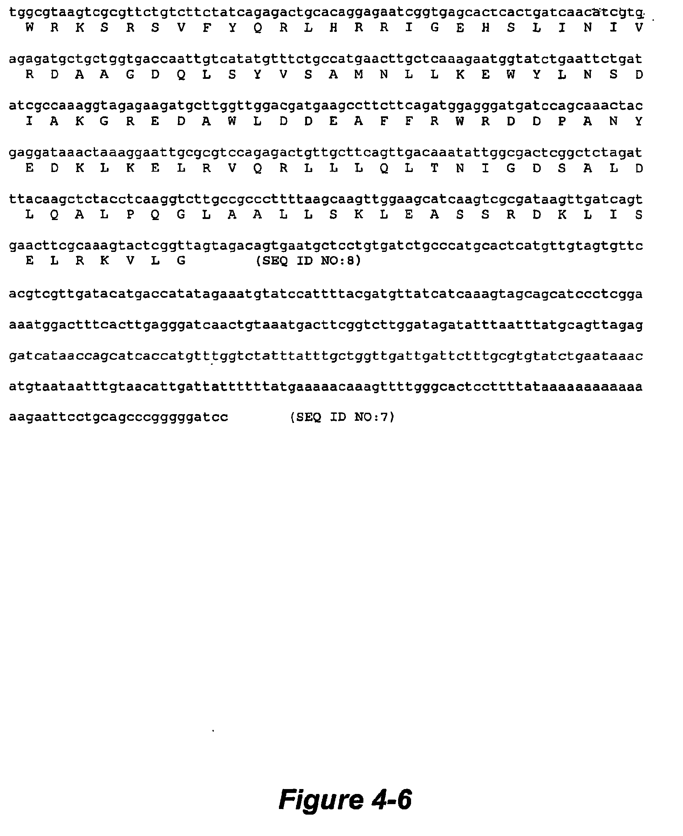

*Figure 4* is the nucleotide and amino acid sequence (SEQ ID NO:7 and 8, respectively) of an alfalfa cytosolic acetyl coA-carboxylase (ACCase) (GenBank Accession No. L25042 plus additional 3' untranslated sequences).

Like reference symbols in the various drawings indicate like elements.

## DETAILED DESCRIPTION

All percent oil content and percent protein content are reported based upon dry weight. As used herein, "oil content" or "percent oil content" refers to the oil content in a particular tissue. "Oils" are typically triacylglycerols. Oil content can be measured in by NMR (using American Oil Chemists' Society (AOCS) Method AM 2-93 and AOCS Recommended Practice AK 4-95) or by NIR (using AOCS Method AK 3-94 and AOCS Procedure AM 1-92).

As used herein, "protein content" or "percent protein content" refers to the protein content in a particular tissue. The protein content in seeds typically includes storage proteins, as well as other peptide/polypeptide components. Protein content can be determined by NIR (using AOCS Method BA 4e-93).

As used herein, "high oleic acid" refers to an oleic acid ($C_{18:1}$) content in seeds greater than 70% based on total fatty acid composition after hydrolysis. A typical high oleic *Brassica* line exhibits an oleic acid content of at least 70%; for example, an oleic acid content of about 80%, or about 90% based on total fatty acid composition after hydrolysis.

Oleic acid is typically measured by gas chromatography (GC) using AOCS Method Ce 1e-91.

As used herein, "high erucic acid" refers to an erucic acid ($C_{22:1}$) content greater than 45% based on total fatty acid composition after hydrolysis. A typical high erucic acid *Brassica* line would exhibit an erucic acid content of at least 45%; for example, an erucic acid content of 50%, 55% or even greater based on total fatty acid composition after hydrolysis. Erucic acid is typically measured by GC using AOCS Method Ce le-91.

As used herein, "FDA saturated fatty acid content" is the total of myristate ($C_{14:0}$), palmitate ($C_{16:0}$), stearate ($C_{18:0}$), arachidate ($C_{20:0}$), behenate ($C_{22:0}$) and lignocerate ($C_{24:0}$). Fatty acid profiles reported herein were obtained by GC (using AOCS Method Ce le-91).

As used herein, a "variety" is a group of plants that display little or no genetic variation between individuals for at least one trait. Varieties may be created by, e.g., several generations of self-pollination and selection, or vegetative propagation from a single parent using tissue or cell culture techniques.

As used herein, a "line" refers to a plant and its progeny produced from a single transformation.

*Nucleic acid constructs*

A nucleic acid construct useful in the invention comprises a multi-functional cytosolic acetyl coA-carboxylase (ACCase) coding sequence operably linked to a promoter. Suitable cytosolic ACCases include plant and animal cytosolic ACCases from organisms such as *Arabidopsis thaliana (e.g.,* GenBank Accession No. L27074), *Brassica napus (e.g.,* GenBank Accession No. X77576), *Zea mays (e.g.,* GenBank Accession No. A25273) and *Homo sapiens (e.g.,* GenBank Accession No. U19822). For example, a construct can contain a 35S cauliflower mosaic virus (CaMV) promoter and an alfalfa *(i.e., Medicago sativa)* cytosolic ACCase cDNA *(e.g.,* GenBank Accession No. L25042).

Alternatively, a construct of the invention can contain ACCase nucleic acid sequences from *Saccharomyces cerivisiae (e.g.,* GenBank Accession No. M92156), *Schizosaccharomyces pombe (e.g.,* GenBank Accession No. D78169), *Ustilago maydis* (*e.g.*, GenBank Accession No. Z46886), *Bos taurus* (bovine) (*e.g.*, GenBank Accession No. AJ132890), *Rattus norvegicus* (rat) *(e.g.,* GenBank Accession No. AB004329), Ovis *aries* (sheep) (*e.g.*, GenBank Accession No. X80045), *Gallus gallus* (chicken) *(e.g.,* GenBank Accession No. J03541), *Glycine max* (soybean) (*e.g.*, GenBank Accession No. L42814), *Avena sativa* (oat) *(e.g.,* GenBank Accession No. AF072737), *Triticum aestivum* (wheat) (*e.g.*, GenBank Accession No. U39321) or *Phaseolus vulgaris* (bean) (*e.g.*, GenBank Accession No. AF007803). A representative cloning strategy for producing a construct of the present invention is described herein. Other suitable methods for engineering constructs are described elsewhere, e.g., Sambrook et al., Molecular Cloning: A Laboratory Manual, 2nd ed., Cold Spring Harbor Laboratory Press (1989).

As used herein, "promoter" refers to nucleic acid sequences that, when operably linked to an ACCase coding sequence, direct transcription of the coding sequence such that it's gene product can be produced. Promoters can be described based on their activity (*e.g.*, constitutive, inducible, tissue-specific or temporal-specific). Constitutive promoters are generally nucleic acid sequences that direct a relatively high level of transcription, and typically without great tissue- or temporal-specificity. Inducible promoters are typically nucleic acid sequences that regulate transcription in response to a stimulus (*e.g.*, a physical or chemical stimulus). Tissue- or temporal-specific promoters are generally nucleic acid sequences that direct transcription that is biased toward a particular tissue or time (*e.g.*, a particular developmental stage), respectively. Oftentimes, however, a promoter's activity does not fall under a single description.

Suitable promoters are known (*e.g.*, Weising et al., Ann. Rev. Genetics 22:421-478 (1988)). The following are representative promoters suitable for use in the invention described herein: regulatory sequences from fatty acid desaturase genes (*e.g., Brassica fad2D* or *jad2F,* see WO 00/07430); alcohol dehydrogenase promoter from com; light inducible promoters such as the ribulose bisphosphate carboxylase (Rubisco) small subunit gene promoters from a variety of species; major chlorophyll a/b binding protein gene promoters; the 19S promoter of cauliflower mosaic virus (CaMV); a seed-specific promoter such as a napin or cruciferin seed-specific promoter; as well as synthetic or other natural promoters that are, for example, inducible, constitutive, tissue-specific or temporal-specific.

A nucleic acid encoding a cytosolic ACCase may or may not contain introns within the coding sequence. Introns are nucleic acid sequences that are initially transcribed into RNA and subsequently removed. The number of introns in a transcript can vary, as can the size of each intron. Introns themselves possess very little conservation, but the splice site sequences *(i.e.,* the sequence at the exon-intron and intron-exon junctions) typically are highly conserved among eukaryotes. In addition, introns typically possess an internal conserved sequence corresponding to an branch site involved in intron removal. Nucleic acid sequences containing an ACCase open reading frame can be examined for introns using, for example, software such as the Sequence Analysis Software Package of the Genetics Computer Group (GCG) (University of Wisconsin Biotechnology Center, 1710 University Avenue, Madison, WI 53705). An ACCase nucleic acid having introns can be, for example, a genomic ACCase nucleic acid (*e.g.*, GenBank Accession No. L27074). An ACCase nucleic acid lacking introns can be, for example, a complementary DNA (cDNA) of an ACCase mRNA nucleic acid (*e.g.,* SEQ ID NO:7), or can be assembled (*e.g.*, recombinantly) from individual exonic sequences.

It should be appreciated that many different nucleic acids will encode a polypeptide having a particular cytosolic ACCase amino acid sequence. The degeneracy of the genetic code is well known in the art, *i.e.,* many amino acids are coded for by more than one nucleotide codon. It should also be appreciated that certain amino acid substitutions can be made within polypeptide sequences without affecting the function of the polypeptide. Conservative amino acid substitutions or substitutions of similar amino acids often are tolerated without affecting polypeptide function. Similar amino acids can be those that are similar in size and/or charge properties. Similarity between amino acids has been assessed in the art. For example, Dayhoff et al. (1978) in Atlas of Protein Sequence and Structure, Vol. 5, Suppl. 3, pp. 345-352, incorporated herein by reference, provides frequency tables for amino acid substitutions that can be employed as a measure of amino acid similarity.

Additional regulatory sequences may be useful in the nucleic acid constructs of the present invention, including, but not limited to, polyadenylation sequences, enhancers, introns, and the like. Such elements may not be necessary for expression of the ACCase coding sequence, although they may increase expression by affecting transcription, stability of the mRNA, translational efficiency, or the like. Such elements can be included in a nucleic acid construct as desired to obtain optimal expression of the ACCase nucleic acid in the host cell(s). Sufficient expression, however, may sometimes be obtained without such additional elements. A representative reference describing certain regulatory elements is Weising et al., Ann. Rev. Genetics 22:421-478 (1988).

*Transgenic plants*

In one aspect of the invention, transgenic plants are created by introducing an ACCase nucleic acid construct into a plant cell and growing the plant cell into a plant. Such plants contain and express the ACCase nucleic acid construct. Suitable techniques for introducing nucleic acids into plant cells to create such plants include, without limitation, *Agrobacterium*-mediated transformation, viral vector-mediated transformation, electroporation and particle gun transformation. Illustrative examples of transformation techniques are disclosed in U.S. Patent 5,204,253, (describing biolistic transformations), U.S. Patent 6,051,756 (describing biolistic transformation of *Brassica)* and U.S. Patent 5,188,958 (describing *Agrobacterium* transformation). Transformation methods utilizing the Ti and Ri plasmids of *Agrobacterium spp.* typically use binary-type vectors (*e.g.,* ptet1, pBinl9) (Walkerpeach et al., in Plant Molecular Biology Manual, Gelvin & Schilperoort, eds., Kluwer Dordrecht, C1:1-19 (1994)).

Techniques are known for the introduction of DNA into dicots as well as monocots, as are the techniques for culturing such tissues and regenerating plants. If cell or tissue cultures are used as the recipient tissue for transformation, plants can be regenerated from transformed cultures by techniques known to those skilled in the art. Suitable dicots include plants such as alfalfa, soybean, rapeseed (high erucic and canola), and sunflower. Monocots that have been successfully transformed and regenerated in the art include wheat, corn, rye, rice, sorghum and asparagus (see, e.g., U.S. Patent Nos. 5,484,956 and 5,550,318).

Preferred species for generating transgenic plants of the present invention include, without limitation, oil-producing species, such as soybean *(Glycine max),* rapeseed *(e.g., Brassica napus, B. rapa* and *B. juncea)* (both Spring and Winter maturing types within each species), sunflower *(Helianthus annus),* castor bean *(Ricinus communis),* safflower *(Carthamus tinctorius),* palm *(e.g., Elaeis guineensis),* coconut *(e.g., Cocos nucifera),* meadowfoam *(e.g., Limnanthes alba alba* and *L. douglasii),* cottonseed *(e.g., Gossypium hirsutum),* olive *(e.g., Olea europaea),* peanut *(e.g., Arachis hypogaea),* flax *(e.g., Linum usitatissimum),* sesame *(e.g., Sesamum indicum)* and crambe *(e.g., Crambe abyssinica* or *C. hispanica).* Accordingly, suitable families include, but are not limited to, *Solanaceae, Leguminaceae, Brassicaceae* and *Asteraceae.* A transgenic plant of the invention typically is a member of a plant variety within the families or species mentioned above.

As used herein, a transgenic plant also refers to progeny of an initial transgenic plant. Progeny includes descendants of a particular plant or plant variety, e.g., seeds developed on a particular plant. Progeny of a plant also includes seeds formed on $F_1$, $F_2$, $F_3$, and subsequent generation plants, or seeds formed on $BC_1$, $BC_2$, $BC_3$, and subsequent generation plants. Seeds produced by a transgenic plant can be grown and then selfed (or out-crossed and selfed) to obtain plants homozygous for the construct. Seeds can be analyzed to identify those homozygotes having the desired level of expression of a construct. Alternatively, transgenic plants and progeny thereof may be obtained by vegetative propagation of a transformed plant cell (for those species amenable to such techniques).

Transgenic plants can be used in commercial breeding programs for the species of interest or can be crossed or bred to plants of related crop species. Phenotypes conferred by expression of an ACCase nucleic acid construct can be transferred from one species to another species by, for example, protoplast fusion. Such breeding programs are useful to incorporate other agronomic or specialty traits of interest, e.g., herbicide tolerance or a high oleic acid content in seeds.

*Methods*

In one aspect of the invention, there are provided methods of generating a plant that produces seeds exhibiting a statistically significant increase in oil content. The method includes introducing a nucleic acid construct containing a promoter and an ACCase coding sequence into a plant and selecting progeny that produce seeds with increased oil content as compared to seeds from a corresponding plant lacking the ACCase nucleic acid construct, e.g., seeds from a plant having the same or similar genetic background as the transgenic plant but which does not have the cytosolic ACCase construct. Such progeny are identified after one or more generations of selection, *e.g.,* one generation, three or more generations, or six or more generations. By way of example, selection may be carried out initially, e.g., the first and second generations, by selecting those progeny possessing the ACCase construct, and selection in subsequent generations may be carried out by identifying those progeny that possess the ACCase construct as well as elevated seed oil content.

Also provided by the invention are methods of producing seeds with a statistically significant increase in oil content. The methods include introducing a nucleic acid construct containing a promoter and an ACCase coding sequence into one or more plant cells and regenerating such plant cells into one or more plants. Seeds exhibiting statistically significantly increased oil content can then be harvested from selected progeny of the plant.

Further provided by the invention are methods of increasing the oil content in seeds. The methods include introducing a nucleic acid construct containing a promoter and a cytosolic ACCase coding sequence into a plant and selecting progeny after at least one generation of selection that produce seed with increased oil content as compared to corresponding seeds produced from plants lacking the recombinant ACCase nucleic acid.

The following Table provides relative percent oil and protein content on a dry weight basis (unless indicated otherwise) in several plants, particularly oilseed plants, that can be used in the present invention.

| Plant | % Oil | % Protein | Key |
|---|---|---|---|
| Soybean *(Glycine max)* | -20 | ~40 | c |
| Rapeseed *(Brassica napus)* | 40-44 | 38-41 (oil free meal) | c; d |
| Sunflower *(Helianthus annus)* | 40 | | d |
| Castor bean *(Ricinus communis)* | 50 | | a |
| Safflower *(Carthamus tinctorius)* | 36.8-47.7 | 15.4-22.5 | d |
| Crambe *(Crambe abyssinica)* | 30-35 | ~28 | b |
| Palm *(Elaeis guineensis)* | 20<br>>50 | | c; per fresh fruit bunch (~20% moisture); dried kernels |
| Coconut *(Cocos nucifera)* | 34<br>69 | 3.5 | d; coconut flesh (50% moisture); dried kernels |
| Maize *(Zea mays)* | 3.1-5.7 | 6-12 | c; d |
| Cottonseed *(Gossypium hirsutum)* | 25-30 | 25-30 | d; kernel |
| Olive *(Olea europaea)* | 19.6 | 1.6 | fruit (52.4% moisture) |
| Peanut *(Arachis hypogaea)* | 36-56 | 25-30 | c; (unknown moisture) |
| Flax *(Linum usitatissimum)* | 35-45 | | d; per fruit capsule (~10 seeds/fruit) |
| Sesame | 53.3- | 25-30 | d; (5-7% moisture) |

(continued)

| Plant | % Oil | % Protein | Key |
|---|---|---|---|
| *(Sesamum indicum)* | 57.5 | | |
| (a) Brigham RD, 1993, Castor: Return of an old crop, p 380-3. In New Crops, Janick, J & Simon, JE, eds. Wiley, NY.<br>(b) Grombacher et al., Cooperative Extension, Institute of Agriculture and Natural Resources, University of Nebraska-Lincoln, *Crambe production,* Publication G93-1126A, G1126 (Field Crops), F-17 (Misc. Crops); see also pubs@unlvm.unl.edu<br>(c) In *Principles of Cultivar Development,* 1987, Fehr, WR, ed., Macmillan Publishing Co., NY.<br>(d) In 5th Edition Bailey's Industrial Oil & Fat Products, Vol. 2, Edible Oil & Fat Products: Oils and Oil Seeds, 1996, Hui, YH, ed., Wiley, NY. | | | |

The present invention describes a novel method of making plants that produce seeds with a statistically significant increase in oil content. As used herein, "statistically significant" refers to a *p*-value of less than 0.05, e.g., a *p*-value of less than 0.025 or a *p*-value of less than 0.01, using an appropriate measure of statistical significance, e.g., a one-tailed two sample t-test

Plants of the invention produce seeds that exhibit an increase in oil content that is statistically significant relative to seeds produced by plants that lack a cytosolic ACCase construct. Plants produced by the method of the present invention produce seeds having an increase in oil of from about 5% to about 25% over the oil content in seeds produced by untransformed control plants. For example, the increase in oil content for plants described herein is from about 5% to about 20%, or from about 5% to about 15%, or from about 10% to about 20%, relative to plants that lack a cytosolic ACCase construct.

Seeds and plants of plant varieties made from the transgenic plants described herein are included within the scope of the invention, as well as progeny of these varieties that possess the novel characteristics recited herein.

Nucleic acid constructs, plants and methods described herein provide for more efficient production of oil for food and industrial applications (*e.g.*, engine lubricants, hydraulic fluids, etc.). For example, plants described herein produce a greater yield of oil per acre planted compared to plants lacking a cytosolic ACCase construct In addition, there is increased oil yield during the processing of such seeds.

The invention will be further described in the following examples, which do not limit the scope of the invention described in the claims.

## EXAMPLES

Example 1-Constructs

The pSP72 vector (Promega) was digested with *Xho*I and *Sal*I and subsequently religated to remove the *Pvu*II site. This modified vector was designated ModpSP72. The tobacco small subunit Rubisco (ssRubisco, also known as ribulose 1,5-bisphosphate carboxylase) transit peptide was amplified by PCR from a tobacco ssRubisco gene/pet11d template using a 5' primer (5'-CAUCAUCAUCAUATCGATAGGTACCAAAAAAAA CAACCATGGCTTCCTCAGTTCTT) (SEQ ID NO:1) and a 3' primer (5'-CUACUAC UACUAGCTAGCCATGGACT TCTTGTTAATTGGTGGCCA) (SEQ ID NO:2). The 5' primer was designed to contain *Cla*I*, KpnI,* and *Nco*I sites, and the 3' primer was engineered to contain *Nco*I and *Nhe*I sites. The amplified transit peptide DNA was annealed into the pAMP1 vector (Gibco BRL) and both strands were sequenced to confirm fidelity. This construct was designated +Transit/pAMP1. To generate a construct lacking the transit peptide (-Transit/pAMP), +Transit/pAMP1 was digested with *Nco*I and religated.

The cloning of a fragment designated 209/180 from an alfalfa cytosolic acetyl coA-carboxylase (ACCase) into the pAMP1 vector to produce 209/180/pAMP1 is described in Shorrosh et al. (1994, Proc. Natl. Acad. Sci. USA, 91: 4323-27). A fragment designated 147/136 was PCR amplified using primers 147 and 136 (Shorrosh et al., 1994), which was subsequently subcloned into the pAMP1 vector to generate a 147/136/pAMP1 construct. The 209/180 fragment was removed from the pAMP1 vector by digesting with *KpnI* and *Bam*HI and subcloned into the *Kpn*I*IBam*HI sites of ModpSP72 to generate a 209/180/pSP72 construct. The 147/136/pAMP1 construct was digested with *SnaB*I and *Bam*HI and the insert containing the 147/136 fragment was subcloned into the *SnaB*I/*Bam*HI sites of the 209/180/pSP72 construct to generate a 209/136/pSP72 construct.

Clone "T1", corresponding to a partial alfalfa ACCase cDNA and described in Shorrosh et al., 1994, was digested with *Pvu*II and *Bam*HI and subcloned into the 209/136/pSP72 construct at the *Pvu*II/*Bam*HI sites to generate 209-T/pSP72. Additionally, a clone designated 3X, corresponding to a partial alfalfa ACCase cDNA (essentially the M2 fragment as described in Shorrosh et al., 1994, with additional 5' and 3' flanking sequences to facilitate cloning), was digested with *Eco*R47III and *Bam*HI and subcloned into the 209-T/pSP72 construct at the *Eco*R47III/*Bam*HI sites to generate 209-3X/pSP72. This construct contains a full-length alfalfa cytosolic ACCase cDNA coding sequence in the pSP72 vector.

The 209-3X/pSP72 construct was digested with *Kpn*I and *Bam*HI and subcloned into the +Transit/pAMPI construct at the *Kpn*I/*Bam*HI sites to generate a construct designated +6ACCase/pAMPI. +6ACCase/pAMP1 contains a full-length alfalfa ACCase cDNA with a transit peptide at the 5' end in the same reading frame as the ACCase coding sequence. The +6ACCase/pAMPI construct was then digested with *Nhe*I and *Bam*HI and the full-length alfalfa ACCase cDNA, including the transit peptide, was subcloned into the *Agrobacterium* binary vector, ptet1 (provided by Dr. C. Gatz, Institute fur Genbiologische, Berlin), at the *Nhe*I/*Bam*HI sites adjacent to the cauliflower mosaic virus (CaMV) 35S promoter. This manipulation generated +6ACCase/ptetl. Similarly, the 209-3X/pSP72 construct was digested with *NheI*-*Bam*HI and the full-length alfalfa ACCase cDNA was subcloned into the -Transit/pAMPI construct at the *Nhe*I/*Bam*HI sites to generate -7ACCase/pAMPI. The -7ACCase/pAMPI construct was then digested with *Kpn*I and *Bam*HI and the full-length alfalfa ACCase cDNA was subcloned into the ptet1 binary vector at the *Kpn*I/*Bam*HI sites adjacent to the CaMV 35S promoter to produce -7ACCase/ptet1. The -7ACCase/ptet1 construct contains a full-length alfalfa ACCase cDNA but lacks a transit peptide.

Example 2-Transgenic plants

The +6ACCase/ptetl and -7ACCase/ptetl constructs of Example 1 were used to transform *Agrobacterium* LBA4404. The resulting *Agrobacterium* transformants were each co-cultivated separately with *B. napus* hypocotyls and cultured consecutively on incubation, selection (containing kanamycin) and regeneration media until green shoots were produced. Regenerated plantlets were transferred to the greenhouse and grown to maturity. Each T1 plant (N=240) was selfed and the resulting T2 seeds were harvested from each individual T1 plant.

The ACCase constructs were introduced into *B. napus* hypocotyls of three different canola varieties as follows. A construct designated -7ACCase was introduced into Westar, a canola variety registered in Canada; and a construct designated +6ACCase was introduced into Oscar, a canola variety registered in Australia (App. No. 1992/009, 19 June, 1996) or IMC 03, a Cargill proprietary low linolenic acid canola variety. Table 1 shows a typical fatty acid profile for each of the *Brassica* varieties used in the transformations.

TABLE 1

| Typical fatty acid profile of Westar, Oscar and IMC 03 seeds | | | |
|---|---|---|---|
| Fatty acid | Westar | Oscar | IMC 03 |
| $C_{16:0}$ | 3.7[1] | 3.7 | 3.9 |
| $C_{16:1}$ | 0.1 | 0.1 | 0.1 |
| $C_{18:0}$ | 2.5 | 2.5 | 1.95 |
| $C_{18:1}$ | 65.0 | 60 | 65.6 |
| $C_{18:2}$ | 17.6 | 22.0 | 18.0 |
| $C_{18:3}$ | 8.0 | 10.0 | 3.00 |
| $C_{20:0}$ | 0.5 | 0.5 | 0.5 |
| $C_{20:1}$ | 1.3 | 1.3 | 1.5 |
| $C_{20:2}$ | 0.1 | 0.1 | 0.1 |
| $C_{22:0}$ | 0.1 | 0.1 | 0.1 |
| $C_{22:1}$ | 0.1 | 0.1 | 0.05 |
| $C_{24:0}$ | 0.1 | 0.1 | 0.1 |
| $C_{24:1}$ | 0.1 | 0.1 | 0.1 |
| FDA | 6.9 | 6.9 | 6.55 |
| % Oil | 45.0 | 41.0 | 46.0 |

(continued)

| Typical fatty acid profile of Westar, Oscar and IMC 03 seeds | | | |
|---|---|---|---|
| Fatty acid | Westar | Oscar | IMC 03 |
| % Protein | 26.97[2,3] | - | 22.46[3] |
| [1]percent; [2]from Principles in Cultivar Development, Vol. 2, Crop Species, W.R. Fehr, ed., pp. 443; [3]percent protein content was estimated based upon the percent protein content reported for the air-dried, oil-free seed meal using the oil content reported and assuming a 5% moisture content; --, Unknown. | | | |

Example 3-Preparation of fatty acid methyl esters and fatty acid analysis of seed by capillary gas liquid chromatography (GLC)

The following describes a means for quantifying fatty acid composition in canola seed. To prepare samples, approximately 150 mg of seed is placed into a 15 ml polypropylene centrifuge tube. The seed is broken apart and 0.6 ml of methanolic KOH solution is added to the tube. After mixing on a vortex mixer for approximately 30 sec, the tube is placed in a water bath at 60°C for 60 sec. About 4.0 ml of saturated NaCl solution is added to the tube followed by 1.0 ml of iso-octane and the sample mixed on a vortex mixer for an additional 30 sec. The sample is centrifuged for 5 min to separate and purify the organic layer. Approximately 700 μl of the organic layer, which contains the fatty acid methyl esters, is removed from the tube and placed into a GC autosampler vial. The vial is purged with nitrogen gas to remove the oxygen and preserve the sample.

Samples are analyzed, based on AOCS Method Ce le-91, by injecting 1.0 μl into a Hewlett Packard 6890 gas chromatograph by means of an autosampler. A normalized percentage is calculated and reported for each fatty acid in the sample.
The GC conditions are as follows:

Column: 5 m x 0.32 mm DB-Wax (0.5 μm film thickness);
Detector: FID;
Inlet temp.: 250°C;
Detector temp.: 250°C;
Split ratio: 100:1;
Carrier gas: helium at 30.0 ml/min; and
Oven program: 1.0 min at 220°C; 10°C/min up to 245°C; and 3.0 min at 245°C.

Example 4-Determination of oil and moisture content in canola seed by NMR spectroscopy

The following is a non-destructive method for determining oil and moisture content in samples of canola seed by means of nuclear magnetic resonance spectroscopy. An Oxford MQA6005 NMR Analyzer (Oxford Analytical Instruments Limited, Concord, MA) is calibrated according to manufacturer specifications. Six samples of canola seed (~22 g/sample) are used for calibration. Samples are selected to represent the oil and moisture ranges over which most unknown samples are expected to fall. The oil content of each sample is determined by Soxhlet extraction (based on AOCS Method Am 2-93). Moisture content is determined by gravimetric means (based on AOCS Method Ai 2-75). The response of each sample is then measured on the NMR instrument. Two calibration curves (one for oil and one for moisture) are prepared using the data collected.

Samples containing unknown amounts of oil and moisture are analyzed according to the instrument manufacturer instructions (based on AOCS Recommended Practice Ak 4-95). The response of each sample is collected and stored by a computer. The results are calculated and expressed as "Oil %", "Moisture %", and "Oil % Normalized to Dry Mass" (conversion from Oil % (as is) to Oil % on a dry basis is calculated using the following formula: Oil % (dry) = Oil % (as is)/[1-(Moisture %/100)]).

Example 5-Determination of percent oil, moisture, protein, chlorophyll, and fatty acids by NIR spectroscopy

The following method provides a means of predicting the levels of oil, moisture, protein, chlorophyll, oleic acid ($C_{18:0}$), linoleic acid ($C_{18:1}$), and linolenic acid ($C_{18:2}$) in canola seed samples by means of near infra-red reflectance spectroscopy.

A Foss NIR Systems model 6500 Feed and Forage Analyzer (Foss North America, Eden Prairie, MN) is calibrated according to the manufacturer's recommendations. Canola seed samples, which represented wide ranges of the sample constituents listed above, are collected for calibration. Lab analysis results are determined using accepted methodology (*i.e.*, oil, AOCS Method Ak 3-94; moisture, AOCS Method Ai 2-75; fatty acid, AOCS Method CE le-91 and AOCS Method CE 2-66; chlorophyll, AOCS Method CC 13D-55; protein, AOCS Method BA 4e-93; and glucosinolates, AOCS Method Ak 1-92). Instrument response is also measured for each sample. A calibration equation is calculated for each constituent by means of chemometrics. These equations are combined into one computer file and are used for prediction of the constituents contained in unknown canola samples.

Seed samples containing unknown levels of the above constituents are prepared by removing foreign material from the sample. Cleaned whole seed is placed into the instrument sample cell and the cell is placed into the instrument sample assembly. Analysis is carried out according to instrument manufacturer instructions (based on AOCS Procedure Am 1-92). The results are predicted and reported as % constituent (% oil and protein are reported based on dry weight). Conversion from 'dry weight' basis to 'as is' basis for oil and protein is calculated using the following formula:

$$\text{constituent (as is)} = \text{constituent (dry wt.)} \times [1-(\%\ \text{moisture}/100)].$$

Example 6-T1 plants and T2 seeds

A total of 126 -7ACCase/Westar plants were regenerated in a greenhouse from the plantlets described in Example 2. Each T1 plant was selfed and a sample of T2 seeds from each plant was analyzed for fatty acid composition by gas chromatography as described in Example 3. T2 seeds had fatty acid compositions that were not significantly different from the fatty acid profile of the Westar background variety.

Table 2 shows the mean fatty acid profile (± standard deviation) for the -7ACCase/ Westar transformation. T2 seeds from each T1 plant were advanced (*i.e.,* no selection was performed on T2 seeds) such that 5-10 seeds from each T1 plant were grown individually in a single row in the greenhouse.

TABLE 2

| Mean fatty acid profile of T2 seeds | |
|---|---|
| Fatty acid | -7ACCase/Westar |
| $C_{14:0}$ | 0.07 (0.04)[1] |
| $C_{16:0}$ | 4.59 (0.69) |
| $C_{16:1}$ | 0.31 (0.13) |
| $C_{18:0}$ | 2.28 (0.45) |
| $C_{18:1}$ | 61.61 (3.53) |
| $C_{18:2}$ | 20.18 (2.27) |
| $C_{18:3}$ | 7.95 (1.04) |
| $C_{20:0}$ | 0.77 (0.12) |
| $C_{20:1}$ | 1.23 (0.10) |
| $C_{20:2}$ | 0.08 (0.02) |
| $C_{22:0}$ | 0.45 (0.09) |
| $C_{22:1}$ | 0.03 (0.03) |
| $C_{24:0}$ | 0.24 (0.10) |
| $C_{24:1}$ | 0.24 (0.17) |

(continued)

| Mean fatty acid profile of T2 seeds | |
|---|---|
| Fatty acid | -7ACCase/Westar |
| FDA | 8.39 (1.22) |
| [1]mean in percent (± standard deviation) | |

Table 3 shows fatty acid profiles of T2 seeds from representative individual lines from the -7ACCase/Westar transformation.

TABLE 3

| Fatty acid profile of T2 seeds from representative individual -7ACCase/Westar *Brassica* lines | | | | |
|---|---|---|---|---|
| | 001-01[1] | 001-120 | 001-121 | 001-31 |
| $C_{14:0}$ | 0.103[2] | 0.448 | 0.154 | 0.056 |
| $C_{16:0}$ | 7.720 | 7.053 | 6.339 | 4.222 |
| $C_{16:1}$ | 0.776 | 0.821 | 0.820 | 0.249 |
| $C_{18:0}$ | 3.181 | 3.107 | 4.213 | 1.988 |
| $C_{18:1}$ | 55.584 | 51.827 | 50.624 | 64.292 |
| $C_{18:2}$ | 22.659 | 24.794 | 25.949 | 18.319 |
| $C_{18:3}$ | 6.806 | 8.343 | 7.041 | 8.106 |
| $C_{20:0}$ | 1.170 | 0.969 | 1.235 | 0.688 |
| $C_{20:1}$ | 0.950 | 0.815 | 0.902 | 1.337 |
| $C_{20:2}$ | 0.000 | 0.065 | 0.082 | 0.065 |
| $C_{22:0}$ | 1.170 | 0.578 | 0.912 | 0.381 |
| $C_{22:1}$ | 0.000 | 0.024 | 0.188 | 0.000 |
| $C_{24:0}$ | 0.379 | 0.421 | 0.574 | 0.168 |
| $C_{24:1}$ | 0.000 | 0.735 | 0.966 | 0.129 |
| FDA | 13.225 | 12.576 | 13.427 | 7.504 |
| [1]line designation; [2]% | | | | |

Example 7-T2 plants and T3 seeds

A total of 834 T2 plants from 126 lines were selfed and the resulting T3 seed analyzed for fatty acid composition by gas chromatography as described in Example 3.

Table 4 shows summary statistics (mean ± standard deviation) of fatty acid profiles of seeds from the total population of T3 plants produced in the -7ACCase/Westar transformation, from those T3 plants selected for advancement and from plants corresponding to the non-transgenic Westar variety. Data for the non-transgenic control plants was obtained from 19 Westar plants grown under similar conditions.

TABLE 4

| Mean fatty acid profile of T3 seeds | | | |
|---|---|---|---|
| Fatty acid | -7ACCase/Westar | | |
| | Total[1] | Sel[1] | Control[1] |
| $C_{14:0}$ | 0.06[2] (0.02) | 0.06 (0.03) | 0.06 (0.02) |

(continued)

| Mean fatty acid profile of T3 seeds | | | |
|---|---|---|---|
| Fatty acid | -7ACCase/Westar | | |
| | Total[1] | Sel[1] | Control[1] |
| $C_{16:0}$ | 4.71 (0.79) | 4.90 (1.03) | 4.45 (0.51) |
| $C_{16:1}$ | 0.27 (0.10) | 0.28 (0.13) | 0.23 (0.10) |
| $C_{18:0}$ | 2.84 (0.68) | 3.25 (0.68) | 2.68 (0.52) |
| $C_{18:1}$ | 66.62 (5.34) | 67.20 (6.69) | 69.76 (3.41) |
| $C_{18:2}$ | 15.67 (3.50) | 14.46 (3.88) | 13.78 (2.46) |
| $C_{18:3}$ | 5.68 (1.43) | 5.20 (1.26) | 4.90 (1.05) |
| $C_{20:0}$ | 1.14 (0.26) | 1.32 (0.21) | 1.08 (0.29) |
| $C_{20:1}$ | 1.38 (0.21) | 1.43 (0.24) | 1.41 (0.14) |
| $C_{20:2}$ | 0.07 (0.02) | 0.06 (0.02) | 0.05 (0.02) |
| $C_{22:0}$ | 0.74 (0.22) | 0.87 (0.21) | 0.75 (0.16) |
| $C_{22:1}$ | 0.03 (0.04) | 0.03 (0.04) | 0.02 (0.02) |
| $C_{24:0}$ | 0.50 (0.16) | 0.60 (0.13) | 0.55 (0.15) |
| $C_{24:1}$ | 0.31 (0.13) | 0.34 (0.13) | 0.28 (0.06) |
| FDA | 9.99 (1.72) | 11.00 (1.84) | 9.56 (1.09) |

[1]Total, mean composition of all T2 plants; Sel, mean composition of T2 plants selected for advancement; Control, mean composition of non-transformed Westar plants; [2]mean in percent (± standard deviation).

Three hundred ninety-five plots of T2 plants (representing 104 lines) from the -7ACCase/Westar transformation were selected for advancement based on T3 seeds exhibiting one or more of the following properties in fatty acid composition: $C_{18:0}$ >3.45%, $C_{18:2}$ <13.1%, $C_{18:3}$ <4.51%, $C_{20:0}$ > 1.55%, or FDA saturates (defined as the sum of $C_{14:0}$, $C_{16:0}$, $C_{18:0}$, $C_{20:0}$, $C_{22:0}$ and $C_{24:0}$) >10.5%.

Table 5 shows the fatty acid profile of T3 seed from representative individual lines from the -7ACCase/Westar transformation that were selected for advancement. Bolded numbers indicate criteria used to select and advance the plants.

TABLE 5

| Fatty acid profile of T3 seeds from representative -7ACCase/Westar lines selected for advancement | | | | | | |
|---|---|---|---|---|---|---|
| Fatty acid | 001-26-01[1] | 001-27-12 | 001-30-02 | 001-31-05 | 001-31-07 | 001-78-04 |
| $C_{14:0}$ | 0.0755[2] | 0.1100 | 0.0371 | 0.0393 | 0.0481 | 0.1419 |
| $C_{16:0}$ | 7.3213 | 10.6772 | 3.9496 | 3.8797 | 4.1826 | 10.7381 |
| $C_{16:1}$ | 0.3434 | 0.6230 | 0.1360 | 0.1408 | 0.2025 | 0.6661 |
| $C_{18:0}$ | 4.1630 | 2.9979 | 2.1986 | 2.6323 | 3.0341 | 7.5048 |
| $C_{18:1}$ | 42.6632 | 28.1662 | 72.7333 | 72.5224 | 70.0784 | 31.4668 |
| $C_{18:2}$ | 29.2415 | 37.0096 | 11.5253 | 11.4008 | 12.2550 | 29.4236 |
| $C_{18:3}$ | 9.5494 | 12.4735 | 4.5227 | 4.6344 | 5.1038 | 10.4718 |
| $C_{20:0}$ | 1.7108 | 1.5811 | 1.1132 | 1.2070 | 1.3821 | 2.6041 |
| $C_{20:1}$ | 2.2521 | 2.4504 | 1.7807 | 1.6792 | 1.6406 | 2.6668 |
| $C_{20:2}$ | 0.1620 | 0.1600 | 0.0726 | 0.0724 | 0.0700 | 0.1295 |
| $C_{22:0}$ | 1.1188 | 1.7861 | 0.9534 | 0.9449 | 0.9628 | 1.8995 |
| $C_{22:1}$ | 0.1219 | 0.2030 | 0.1075 | 0.0875 | 0.0000 | 0.0000 |
| $C_{24:0}$ | 0.7574 | 0.9286 | 0.5629 | 0.5129 | 0.6498 | 1.6442 |
| $C_{24:1}$ | 0.5197 | 0.8334 | 0.3071 | 0.2465 | 0.3903 | 0.6428 |
| FDA | 15.1468 | 18.0809 | 8.8148 | 9.2161 | 10.2594 | 24.5325 |
| [1]line designation; [2]% | | | | | | |

Example 8-T3 plants and T4 seeds

About 0.5 g of T3 seed from each T2 plant selected for advancement as described in Example 7, were planted in field plots in Colorado, USA. T4 seeds were collected and combined from 20 random T3 plants from each line and analyzed for fatty acid composition (by GC; see Example 3) and oil content (by NMR; see Example 4). Following random bulk T4 seed analysis from each plot, 5-10 T4 seeds from those lines exhibiting increased oil content were advanced individually in the greenhouse.

Thirteen T3 lines with oil content of 48.7% to 50% were advanced and one T3 line with oil content of 48.1 % was advanced from the -7ACCase/Westar transformation. Table 6 shows summary statistics (mean ± standard deviation) for seed fatty acid profiles of the total T4 population, the plants selected for advancement and corresponding non-transgenic control plants. Data for the non-transgenic control population was obtained from 139 Westar plants transgenic for an *fae1* gene. The *fae1* gene elongates $C_{18:1}$ to $C_{20:1}$, thereby resulting in an accumulation of $C_{20:1}$ in plants transgenic for *fae1,* but does not affect oil content.

TABLE 6

| Mean fatty acid profile and oil content of T4 seeds | | | |
|---|---|---|---|
| Fatty acid | -7ACCase/Westar | | |
| | Total[1] | Sel[1] | Control[1] |
| $C_{14:0}$ | 0.06[2] (0.01) | 0.06 (0.01) | 0.07 (0.03) |
| $C_{16:0}$ | 3.52 (0.40) | 3.52 (0.12) | 3.48 (0.52) |
| $C_{16:1}$ | 0.19 (0.03) | 0.18 (0.01) | 0.19 (0.05) |
| $C_{18:0}$ | 3.00 | 2.12 | 2.15 |

(continued)

| Mean fatty acid profile and oil content of T4 seeds | | | |
|---|---|---|---|
| Fatty acid | -7ACCase/Westar | | |
| | Total[1] | Sel[1] | Control[1] |
| | (8.76) | (0.13) | (0.24) |
| $C_{18:1}$ | 68.58 (6.64) | 69.83 (0.33) | 64.60 (8.61) |
| $C_{18:2}$ | 15.26 (1.98) | 15.30 (0.44) | 14.68 (1.32) |
| $C_{18:3}$ | 6.42 (0.90) | 6.40 (0.29) | 6.49 (0.58) |
| $C_{20:0}$ | 0.71 (0.11) | 0.66 (0.01) | 0.75 (0.18) |
| $C_{20:1}$ | 1.26 (0.19) | 1.18 (0.05) | 5.47 (7.83) |
| $C_{20:2}$ | 0.05 (0.01) | 0.05 (0.00) | 0.14 (0.18) |
| $C_{22:0}$ | 0.34 (0.04) | 0.29 (0.01) | 0.32 (0.04) |
| $C_{22:1}$ | 0.08 (0.44) | 0.05 (0.08) | 0.92 (2.23) |
| $C_{24:0}$ | 0.30 (0.29) | 0.22 (0.04) | 0.22 (0.09) |
| $C_{24:1}$ | 0.24 (0.25) | 0.15 (0.09) | 0.52 (0.57) |
| FDA | 7.92 (8.58) | 6.86 (0.16) | 6.98 (0.65) |
| % Oil | 45.7 (3.2) | 49.1 (0.51) | 45.5 (1.92) |

[1]Total, mean composition of all T3 plants; Sel, mean composition of T3 plants selected for advancement; Control, mean composition of non-transformed Westar plants; [2]mean in percent (± standard deviation).

Table 7 shows the fatty acid profiles of T4 seed from representative individual lines from the -7ACCase/Westar transformation that were selected for advancement.

TABLE 7

| Fatty acid profile and oil content of T4 seeds from representative -7ACCase/Westar lines selected for advancement | | | | |
|---|---|---|---|---|
| Fatty acid | 001-31-07[1] | 001-30-02 | 001-31-06 | 001-30-05 |
| $C_{14:0}$ | 0.06[2] | 0.06 | 0.06 | 0.07 |
| $C_{16:0}$ | 3.59 | 3.52 | 3.42 | 3.70 |
| $C_{16:1}$ | 0.19 | 0.17 | 0.17 | 0.18 |
| $C_{18:0}$ | 2.01 | 2.10 | 2.07 | 2.10 |

(continued)

| Fatty acid profile and oil content of T4 seeds from representative -7ACCase/Westar lines selected for advancement | | | | |
|---|---|---|---|---|
| Fatty acid | 001-31-07[1] | 001-30-02 | 001-31-06 | 001-30-05 |
| $C_{18:1}$ | 69.76 | 69.47 | 70.12 | 69.49 |
| $C_{18:2}$ | 15.64 | 16.21 | 15.08 | 15.49 |
| $C_{18:3}$ | 6.24 | 5.99 | 6.48 | 6.45 |
| $C_{20:0}$ | 0.65 | 0.65 | 0.66 | 0.67 |
| $C_{20:1}$ | 1.13 | 1.14 | 1.18 | 1.15 |
| $C_{20:2}$ | 0.04 | 0.05 | 0.05 | 0.05 |
| $C_{22:0}$ | 0.30 | 0.28 | 0.30 | 0.29 |
| $C_{22:1}$ | 0.02 | 0.02 | 0.02 | 0.01 |
| $C_{24:0}$ | 0.23 | 0.23 | 0.26 | 0.23 |
| $C_{24:1}$ | 0.14 | 0.12 | 0.14 | 0.12 |
| FDA | 6.84 | 6.84 | 6.76 | 7.06 |
| %Oil | 50.0 | 50.0 | 49.4 | 49.4 |
| [1]line designation; [2]% | | | | |

Example 9-T4 plants and T5 seeds

T4 seeds from 10 random selfed plants representing each line selected for advancement in Example 8 were planted in a greenhouse using 5-10 seeds per row. T4 plants were selfed, and T5 seeds were collected from individual plants. A portion of the T5 seeds from each line were combined and analyzed for oil content and fatty acid analysis by NIR as described in Example 5.

Table 8 shows summary statistics (mean ± standard deviation) for seed oil and seed protein content for the total T5 population, for T5 lines selected for advancement and for corresponding non-transgenic controls. Data for the Westar control plants was obtained from 5 'control samples'. Each 'control sample' contained seed bulked from approximately 20 control plants.

Forty-five T4 plants (representing 6 lines from 14 plots) from the - 7ACCase/Westar transformation yielded seed having an oil content of 44.4% to 50.4% and 7 of those plants, representing 2 lines (001-30-02 and 001-31-07) yielding seed having an oil content ranging from 44.4% to 50.1%, were advanced.

TABLE 8

| Oil content, protein content and fatty acid profile ofT5 seeds | | | |
|---|---|---|---|
| Fatty acid | -7ACCase/Westar | | |
| | Total[1] | Sel[1] | Control[1] |
| $C_{18:1}$) | 69.00[2] (0.90) | 69.40 (0.60) | 68.15 (0.56) |
| $C_{18:2}$ | 13.60 (1.30) | 13.20 (29.00) | 14.60 (0.68) |
| $C_{18:3}$ | 7.60 (0.60) | 7.80 (0.30) | 7.09 (0.50) |
| % Oil | 49.90 (1.60) | 49.90 (1.60) | 45.64 (1.22) |
| % Protein | 20.00 (1.10) | 20.50 (1.20) | 24.20 (0.22) |
| Chlor[3] | 36.60 | 37.10 | 18.15 |

(continued)

| Oil content, protein content and fatty acid profile ofT5 seeds | | | |
|---|---|---|---|
| Fatty acid | -7ACCase/Westar | | |
| | Total[1] | Sel[1] | Control[1] |
| | (18.20) | (25.60) | (4.86) |
| Gluc[4] | 4.80<br>(1.00) | 4.60<br>(1.10) | ND<br>ND |
| [1]Total, mean composition of all T4 plants; Sel, mean composition of T4 plants selected for advancement; Control, mean composition of non-transformed Westar plants; [2]mean in percent (± standard deviation); [3]Chlorophyl content reported in parts per million (ppm); [4]Glucosinolate content reported in μmol/g; ND, not determined. | | | |

Table 9 shows the fatty acid profiles of T5 seed from representative individual lines from the -7ACCase/Westar transformation that were selected for advancement.

TABLE 9

| Oil content, protein content and fatty acid profile of T5 seed from representative -7ACCase/Westar lines selected for advancement | | | | |
|---|---|---|---|---|
| | 001-30-02[1] | 001-30-02 | 001-30-02 | 001-31-07 |
| $C_{18:1}$ | 69.0[2] | 69.6 | 70.4 | 69.2 |
| $C_{18:2}$ | 12.6 | 12.5 | 12.3 | 14.1 |
| $C_{18:3}$ | 8.5 | 8.1 | 7.6 | 7.8 |
| % Oil | 50.3 | 50.2 | 49.8 | 52.3 |
| % Protein | 20.9 | 20.3 | 20.6 | 18.4 |
| Chlor[3] | 63.9 | 20.8 | 31.3 | 13.7 |
| Gluc[4] | 5.8 | 4.1 | 4.2 | 3.2 |
| [1]line designation; [2]% | | | | |

Example 10-T5 plants and T6 seeds

T5 lines that were selected based on percent oil and protein content as described in Example 9 were advanced in the field in Colorado, USA and in Saskatchewan, Canada. Approximately 0.5 g of seeds from each selected line were planted and selfed. At maturity, T6 seeds were collected from 20 plants of each line and pooled for analysis of oil content and fatty acid composition by NIR. Based upon NIR analysis and favorable oil content in the pooled sample of T6 seed, T6 seed from 10 random T5 plants from each line were advanced in the greenhouse.

Two lines from the -7ACCase/Westar transformation from T5 plants grown in Canada had T6 seeds that exhibited an oil content of 38.4% to 49.5%. The protein content in seeds harvested from the Canada-grown plants was measured in air-dried, oil-free seed meal (using the 'Generic Combustion Method for Determination of Crude Protein', AOCS Method Ba 4e-93), and the mean was determined to be 46.62% (±1.33) in T6 seed from the -7ACCase/Westar transformation. Percent protein content as shown in Table 10 for the Canadian samples was estimated based upon the percent protein content reported for the air-dried, oil-free seed meal using the oil content reported and assuming a moisture content of 5%.

T5 plants representing six lines of the -7ACCase/Westar transformation grown in Colorado, USA produced seed that had an oil content of 41.9% to 51.0%, and plants from five different lines, having an oil content of 48.8% to 50.5%, were advanced.

Table 10 shows the mean oil content (± standard deviation) of the T6 plants and control plants grown in Canada, and Table 11 shows the corresponding data for the total population ofT6 plants grown in the USA, those T6 plants selected for advancement and from non-transgenic control plants grown in the USA. USA-grown controls for the -7ACCase/Westar transformation consisted of 2 control samples each of IMC129 and IMC130 (IMC129 and IMC130 are both related in the following way to the Westar variety: IMC129 carries a mutation and is otherwise ≥99% Westar background, while IMC130 is the result of a cross between IMC01 and IMC129 varieties, and therefore, contains ≤50% of the Westar background). Canadian-grown controls for the -7ACCase/Wester transformation consisted of 2 IMC 130 control samples.

TABLE 10

Oil content, protein content and fatty acid profile of Canadian-grown T6 seeds

| | -7ACCase/Westar | |
|---|---|---|
| | Total[1] | Control[1] |
| % Oil | 46.78[2] (2.88) | 42.69 (0.18) |
| % Protein | 26.12[3] | 26.66[3] |

[1]Total, mean composition of all T5 plants; Control, mean composition of non-transformed Westar plants; [2]mean in percent (± standard deviation); [3]percent protein content was estimated based upon the percent protein content reported for the air-dried, oil-free seed meal using the oil content reported and assuming a moisture content of 5%.

TABLE 11

Oil content, protein content and fatty acid profile of USA-grown T6 seeds

| | -7ACCase/Westar | | |
|---|---|---|---|
| | Total[1] | Sel[1] | Control[1] |
| $C_{18:1}$ | 68.71[2] (1.64) | 68.54 (0.77) | 68.19 (6.76) |
| $C_{18:2}$ | 15.17 (1.26) | 15.09 (0.78) | 16.03 (5.12) |
| $C_{18:3}$ | 8.05 (0.56) | 8.02 (0.23) | 7.44 (2.36) |
| % Oil | 47.84 (1.45) | 49.48 (0.66) | 48.54 (1.65) |
| % Protein | 25.18 (1.45) | 23.45 (1.09) | 23.84 (1.90) |
| Chloro[3] | -3.59 (2.99) | -5.67 (1.02 | -0.77 (3.35) |
| Gluc[4] | 5.23 (0.64) | 4.87 (0.69) | 5.35 (1.24) |

[1]Total, mean composition of all T6 plants; Sel, mean composition of T6 plants selected for advancement; Control, mean composition of non-transformed Westar plants; [2]mean in percent (± standard deviation); [3]Chlorophyll content reported in parts per million (ppm); [4]Glucosinolate content reported in μmol/g.

Using a one-tailed, two-sample Student's t-test, results from the T6 seeds were evaluated for statistical significance. The average oil content of the total T6 population from Canadian field plots was compared with the average oil content from the corresponding non-transgenic plants grown in Canada, while the T6 population selected for advancement from field plots in the USA was compared with the corresponding USA-grown control population for each line.

The -7ACCase/Westar plants grown in Canada (n=35) had an average oil content that was significantly higher (p<0.1) than that of the control population (n=2).

The -7ACCase/Westar plants grown in the USA and selected for advancement (n=17) had a higher oil content that was statistically significant (p<0.05) compared to the average oil content of the control plants (n=5).

Table 12 shows the percent oil content in T6 seeds from 6 representative individual lines selected for advancement from the -7ACCase/Westar transformation. Control plants grown in the field in Colorado, USA produced seeds that exhibited an average oil content of 44.62 (on a dry weight basis).

TABLE 12

| Oil content of USA-grown T6 seeds from individual -7ACCase/Westar lines selected for advancement | |
|---|---|
| Line | % Oil |
| Tao-001-30-02 | 48.86-49.58 |
| Tao-001-31-02 | 49.93-50.51 |
| Tao-001-56-01 | 48.86-49.42 |
| Tao-001-56-06 | 48.84-50.39 |
| Tao-001-65-08 | 49.10-49.85 |

Example 11-PCR analysis

A nickel size portion of leaf tissue was taken at 2.5 weeks post-germination from 12 T7 plants (representing 12 different -7ACCase/Westar transformed lines) grown from the T6 seeds described in Example 10. Tissue samples were dried in a food dehydrator at 135°C for 8-16 hrs. DNA was isolated using the Qiagen Dneasy 96 Plant Kit and resuspended in 150 μl buffer.

PCR amplification was performed in a volume of 20 μl containing the following: 1X PCR Buffer containing 1.5 mM $MgCl_2$ (Qiagen PCR Core Kit); 0.2 mM dNTP; 0.5 units Taq polymerase (Qiagen); 0.5 μM MF-ACCase 119 primer (5'-GTAGGCACCCTGCTACTACA (SEQ ID NO:9)); 0.5 μM MF-ACCase 645 primer (5'-CATCAGGAATAGTAATCAAGTCA (SEQ ID NO:10)); 0.4% sucrose; 0.008% Cresol. A 30 cycle amplification was performed using the following PCR conditions:

denaturation at 94°C for 30 sec, annealing at 55°C for 30 sec, and extension at 72°C for 60 sec. PCR products were analyzed by 1.2% agarose gel electrophoresis and visualized by ethidium bromide staining. PCR products of the predicted size were detected in all 12 -7ACCase/Westar plants analyzed, indicating the presence of the alfalfa cytosolic ACCase gene in all lines examined.

Example 12-Crosses between the T6 plants

T6 seeds from the selected lines shown in Table 12 were grown in the field in Colorado, USA. Reciprocal crosses were made between the T6 plants derived from the -7ACCase/Westar transformation (lacking a transit peptide) and two T6 plants derived from a +6ACCase/IMC 03 transformation (having a transit peptide). Plants were grown to maturity and the seeds were harvested. F 1 seeds are grown and the resulting plants are allowed to self-pollinate. The resulting F2 progeny seeds are harvested, and the fatty acid profile is determined and oil and protein content are analyzed as described in Examples 3-5. Samples exhibiting a statistically significant increase in oil content are selected for advancement.

Example 13-Outcrosses between the T6 plants and other plant varieties

T6 seeds from the selected lines shown in Table 12 were grown in the field in Colorado, USA. Crosses were made between the T6 plants derived from the -7ACCase/Westar transformation (lacking a transit peptide) or two T6 plants derived from a +6ACCase/IMC 03 transformation (having a transit peptide) and plants of a *Brassica* line exhibiting high oleic acid content. An example of a high oleic acid *Brassica* variety is Q4275, described in PCT 96/20090. F1 seeds are grown and the resulting plants are allowed to self-pollinate. The resulting F2 progeny seeds are harvested, and the fatty acid profile is determined and oil and protein content are analyzed as described in Examples 3-5. Samples exhibiting a statistically significant increase in oil content, as well as high oleic acid content, are selected for advancement.

T6 seeds from the selected lines shown in Table 12 were grown in the field in Colorado, USA. Crosses were made between the T6 plants derived from the -7ACCase/Westar transformation (lacking a transit peptide) or two T6 plants derived from a +6ACCase/IMC 03 transformation (having a transit peptide) and plants of a *Brassica* line exhibiting elevated oil content but lacking an ACCase construct. Examples of *Brassica* varieties exhibiting elevated oil are IMC106RR and IMC107RR, proprietary Cargill *Brassica* lines. The oil content in IMC106RR or IMC107RR is about 46.5-47% and 47.5-48%, respectively, on a dry weight basis. Another example of a *Brassica* line that exhibits elevated oil content is Polo, a non-transgenic variety registered in Canada (Registration # AG012). Polo has an oil content of about 48.5-49.5% on a dry weight basis. F1 seeds were grown in the greenhouse and the resulting plants allowed to self pollinate. The resulting F2 progeny seeds are harvested, and the fatty acid profile is determined and oil and protein content are analyzed as described in Examples 3-5. Seeds exhibiting an oil content that is significantly higher than either parental line are selected for advancement. Progeny plants are allowed to self-pollinate and the seeds analyzed for oil content. Those seeds exhibiting increased oil content are advanced.

T6 seeds from the selected lines shown in Table 12 were grown in the field in Colorado, USA. Crosses were made between the T6 plants derived from the -7ACCase/Westar transformation (lacking a transit peptide) or two T6 plants derived from a +6ACCase/IMC 03 transformation (having a transit peptide) and plants of a *Brassica* line exhibiting high erucic acid content but lacking an ACCase construct. Suitable high erucic acid *Brassica* lines include, for example, Hero (HE101, HEC01), Mercury, Venus or Neptune which have about 45% or more erucic acid (McVetty et al., Can. J. Plant Sci., 76(2):341-342 (1996); Scarth et al., Can. J. Plant Sci., 75(1):205-206 (1995); and Mc Vetty et al., Can J. Plant Sci., 76(2):343-344 (1996)). F1 seeds were grown in the greenhouse and the resulting plants allowed to self-pollinate. The resulting F2 progeny seeds are harvested, and the fatty acid profile is determined and oil and protein content are analyzed as described in Examples 3-5. Seeds exhibiting an oil content that is significantly higher than either parental line are selected for advancement. Progeny plants are allowed to self-pollinate and the seeds analyzed for oil content. Those seeds exhibiting increased oil content are advanced.

Additionally, PCR is used to examine the segregation of the alfalfa ACCase nucleic acid in the progeny of the above-described crosses. After crossing T6 plants derived from a -7ACCase/Westar transformation or T6 plants derived from a +6ACCase/IMC 03 transformation with an appropriate plant *(i.e.,* a plant exhibiting high oil, high oleic acid or high erucic acid), F1 seeds are harvested, grown in the greenhouse and the resulting plants are allowed to self-pollinate. The resulting F2 progeny seeds are harvested, and PCR is performed to detect the presence of the alfalfa ACCase nucleic acid sequences using DNA extracted from the seed. Alternatively, the F2 seeds are grown into mature F3 plants, and PCR is performed, using DNA extracted from the leaves of the plant to detect the presence of the alfalfa ACCase nucleic acid sequences. Representative PCR primers homologous to the alfalfa ACCase are described in Example 11. If PCR amplification indicates the presence of the alfalfa ACCase nucleic acid sequences, oil content is then determined by NMR or NIR as described in Examples 4 and 5. Seeds or plants are subsequently advanced based upon a positive PCR amplification (*i.e.*, the presence of the alfalfa ACCase nucleic acid sequences) and elevated oil content.

## Example 14 - Increased oil content in crushed seeds

T6 seeds of Example 10 are planted, allowed to pollinate, and the resulting seeds are harvested and crushed. The oil content of the crushed seeds is about 5% to about 25% higher than the oil content in a corresponding plant lacking an ACCase construct. The oil is extracted from the crushed seeds as described in, e.g., U.S. Patent No. 5,969,169 or 5,850,026.

Briefly, the seed is cleaned through commercial seed cleaning equipment to remove foreign matter such as weed seeds, plant material, immature seed and other matter. The cleaned seed is crushed and the resulting oil is processed at the Cargill Plant (West Fargo, ND). Greater than 350 tons of seed is crushed using the processing conditions outlined below.

Whole seed is passed through a double roll Bauermeister flaking rolls with smooth surface rolls available from Bauermeister Inc. (Memphis, TN). The roll gap is adjusted so as to produce a flake 0.25 to 0.30 mm in thickness. Flaked seed is conveyed to a five-tray, 8-foot diameter stacked cooker, manufactured by Crown Iron Works (Minneapolis, MN). The flaked seed moisture is adjusted in the stacked cooker to 5.5-6.0%. Indirect heat from the steam heated cooker trays is used to progressively increase the seed flake temperature to 80-90°C, with a retention time of approximately 20-30 minutes. A mechanical sweep arm in the stacked cooker is used to ensure uniform heating of the seed flakes. Heat is applied to the flakes to deactivate enzymes, facilitate further cell rupturing, coalesce the oil droplets and agglomerate protein particles in order to ease the extraction process.

Heated flakes are conveyed to a screw press from Anderson International Corp. (Cleveland, OH) equipped with a suitable screwworm assembly to reduce press out of the oil from the flakes by approximately 70%. The resulting press cake contains a small percentage of residual oil. Crude oil produced from the pressing operation is passed through a settling tank with a slotted wire drainage top to remove the solids expressed out with the oil in the screw pressing operation. The clarified oil is passed through a plate and frame filter to remove the remaining fine solid particles. The filtered oil is combined with the oil recovered from the extraction process before oil refining.

The press cake produced from the screw pressing operation is transferred to a FOMM basket extractor available from French Oil Mill and Machinery Co. (Piqua, OH) where the oil remaining in the cake is extracted with commercial n-hexane at 55°C. Multiple counter-current hexane washes are used to substantially remove the remaining oil in the press cake, resulting in a press cake that contains residual oil in the extracted cake. The oil and hexane mixture (miscella) from the extraction process is passed through a two-stage rising film tube type distillation column to distill the hexane from the oil. Final hexane removal from the oil is achieved by passing the oil through a stripper column containing disk and doughnut internals under 23-26 in Hg vacuum and at 107-115°C. A small amount of stripping steam is used to facilitate the hexane removal. The oil recovered from the extraction process is combined with the filtered oil from the screw pressing operation, resulting in blended crude oil, and is transferred to oil processing.

In the oil processing, the crude oil is heated to 66°C in a batch-refining tank, to which 0.15% food-grade phosphoric acid, as 85% phosphoric acid, is added. The acid serves to convert the non-hydratable phosphatides to a hydratable form, and to chelate minor metals that are present in the crude oil. The phosphatides and the metal salts are removed from the oil along with the soapstock. After mixing for 60 minutes at 66°C, the oil acid mixture is treated with sufficient sodium hydroxide solution to neutralize the free fatty acids and the phosphoric acid in the acid oil mixture. This mixture is heated to 71°C and mixed for 35 minutes. The agitation is stopped and the neutralized free fatty acids, phosphatides, etc. (soapstock) are allowed to settle into the cone bottom of the refining tank for 6 hours. After the settling period, the soapstock is drained off from the neutralized oil.

A water wash is done to reduce the soap content of the oil by heating the oil to 82°C and adding 12% hot water. Agitation of the mixture continues for 10 minutes. The mixture is allowed to settle out for 4 hours, at which time the water is drained off the bottom of the refining vessel. The water washed oil is heated to 104-110°C in a vacuum bleacher vessel maintained at 24-26 in. Hg vacuum. A slurry of the oil and Clarion 470 bleaching clay available from American Colloid Company (Refining Chemicals Division, Arlington Heights, IL) is added to the oil in the vacuum bleacher. This mixture is agitated for 20 minutes before filtering to remove the bleaching clay. The clay slurry addition is adjusted to provide a Lovibond color (AOCS Official Method Cc 136-4) of less than 1.0 red units when the oil is heated to 288°C under atmospheric pressure. Nitrogen is injected into the filtered bleached oil and is maintained under a nitrogen blanket until the oil is deodorized.

Refined and bleached oil is deodorized in a semi-continuous Votator deodorizer tower at a rate of approximately 7,000 pounds per hour. The deodorization temperature is maintained at 265-268°C with a system pressure of 0.3-0.5 mm Hg absolute pressure. Sparge steam is used to strip off the free fatty acids, color bodies, and odor components. Retention time in the deodorizer is generally 30-90 minutes. The deodorized oil is cooled to 45-50° C and nitrogen is injected prior to removal of the vacuum. The deodorized oil is stored under a nitrogen blanket and the resulting deodorized oil analyzed for fatty acid composition.

## OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

**Claims**

1. A transgenic plant which contains and expresses a recombinant nucleic acid construct, said recombinant nucleic acid construct comprising a nucleic acid encoding a cytosolic ACCase operably linked to a promoter, wherein said construct lacks a nucleic acid encoding a transit peptide operably linked to said nucleic acid encoding said cytosolic ACCase, wherein said transgenic plant produces seed that exhibit a relative increase in oil content as compared to seeds produced by a corresponding plant lacking said nucleic acid construct, wherein said relative increase in oil content is from 5% to 25% on a dry weight basis.

2. The plant of claim 1, wherein said nucleic acid encodes a plant cytosolic ACCase.

3. The plant of claim 2, wherein said nucleic acid encodes an alfalfa cytosolic ACCase.

4. The plant of claim 1, wherein said promoter is a cauliflower mosaic virus (CaMV) 35S promoter.

5. The plant of claim 1 or 4, wherein said nucleic acid encoding said cytosolic ACCase lacks introns.

6. The plant of claim 1, wherein said plant is a soybean plant.

7. The plant of claim 1, wherein said plant is a *Brassica* plant.

8. The plant of claim 7, wherein said plant is selected from the group consisting of *Brassica napus, Brassica rapa, Brassica juncea, Brassica carinata, Brasssica nigra* and *Brassica oleracea.*

9. Seeds produced by the plant of claim 1, wherein said seeds comprise said recombinant nucleic acid construct.

10. Progeny of the plant of claim 1, wherein said progeny seeds comprise and express said recombinant nucleic acid construct and wherein said progeny produce seeds that exhibit said increase in oil content.

11. A method of producing a plant, comprising:

(a) transforming a plant with a nucleic acid construct comprising a nucleic acid encoding a cytosolic ACCase operably linked to a promoter, wherein said construct lacks a nucleic acid encoding a transit peptide operably linked to said nucleic acid encoding said cytosolic ACCase; and
(b) selecting, after at least one generation without selection, progeny plants that produce seeds that exhibit a relative increase in seed oil content as compared to seeds produced by a corresponding plant lacking said nucleic acid construct, wherein said relative increase in oil content is from 5% to 25% on a dry weight basis.

12. The method of claim 11, wherein said nucleic acid encodes a plant cytosolic ACCase.

13. The method of claim 12, wherein said nucleic acid encodes an alfalfa cytosolic ACCase.

14. The method of claim 11, wherein said selecting is for at least three generations.

15. The method of claim 11, wherein said promoter is a CaMV 35S promoter.

16. The method of claim 11, wherein said nucleic acid encoding said cytosolic ACCase lacks introns.

17. The method of claim 11, wherein said plant is a *Brassica* plant.

18. The method of claim 11, wherein said plant is selected from the group consisting of *Brassica napus, Brassica rapa, Brassica juncea, Brassica carinata, Brassica nigra* and *Brassica oleracea.*

19. A method of producing a plant, comprising the steps of:

(a) introducing a construct into one or more plants, said construct comprising a nucleic acid encoding a cytosolic acetyl ACCase operably linked to a promoter, wherein said construct lacks a nucleic acid encoding a transit peptide operably linked to said nucleic acid encoding said cytosolic ACCase,

wherein progeny of one or more of said transgenic plants, following at least one generation without selection, produce seeds that exhibit a relative increase in oil content as compared to seeds produced by a corresponding plant lacking said nucleic acid encoding said ACCase, wherein said relative increase in oil content is from 5% to 25% on a dry weight basis

**20.** A method of increasing the oil content in seeds, comprising the steps of:

(a) creating one or more T1 transgenic plants containing and expressing a recombinant nucleic acid construct, said recombinant nucleic acid construct comprising a nucleic acid encoding a cytosolic ACCase operably linked to a promoter; wherein said construct lacks a nucleic acid encoding a transit peptide operably linked to said nucleic acid encoding said cytosolic ACCase; and
(b) selecting T3 or later progeny of said one or more plants that exhibit an relative increase in seed oil content as compared to seeds produced by a corresponding plant lacking said nucleic acid construct, wherein said relative increase in oil content is from 5% to 25% on a dry weight basis.

**21.** The method of claim 20, wherein said selection step comprises selecting progeny that contain said nucleic acid construct.

**Patentansprüche**

**1.** Transgene Pflanze, welche ein rekombinantes Nukleinsäurekonstrukt enthält und exprimiert, wobei das rekombinante Nukleinsäurekonstrukt eine Nukleinsäure umfasst, welche eine cytosolische ACCase kodiert, die funktionell mit einem Promoter verbunden ist, wobei dem Konstrukt eine Nukleinsäure fehlt, die ein Transitpeptid kodiert, welches funktionell mit der die cytosolische ACCase kodierende Nukleinsäure verbunden ist, wobei die transgene Pflanze Samen erzeugt, welche einen relativen Anstieg im Ölgehalt verglichen mit Samen zeigen, die von einer entsprechenden Pflanze erzeugt werden, der das Nukleinsäurekonstrukt fehlt, wobei der relative Anstieg im Ölgehalt von 5% bis 25% auf Basis des Trockengewichts beträgt.

**2.** Pflanze nach Anspruch 1, wobei die Nukleinsäure eine cytosolische Pflanzen-ACCase kodiert.

**3.** Pflanze nach Anspruch 2, wobei die Nukleinsäure eine cytosolische Alfalfa-ACCase kodiert.

**4.** Pflanze nach Anspruch 1, wobei der Promoter ein Blumenkohl-Mosaikvirus (CaMV) 35S-Promoter ist.

**5.** Pflanze nach Anspruch 1 oder 4, wobei der die cytosolische ACCase kodierenden Nukleinsäure Introns fehlen.

**6.** Pflanze nach Anspruch 1, wobei die Pflanze eine Sojabohnenpflanze ist.

**7.** Pflanze nach Anspruch 1, wobei die Pflanze eine Brassica-Pflanze ist.

**8.** Pflanze nach Anspruch 7, wobei die Pflanze aus der Gruppe, bestehend aus *Brassica napus, Brassica rapa, Brassica juncea, Brassica carinata, Brassica nigra* und *Brassica oleracea* ausgewählt ist.

**9.** Samen, erzeugt von der Pflanze nach Anspruch 1, wobei die Samen das rekombinante Nukleinsäurekonstrukt umfassen.

**10.** Nachkommen der Pflanze nach Anspruch 1, wobei die Nachkommen-Samen das rekombinante Nukleinsäurekonstrukt umfassen und exprimieren, und wobei die Nachkommen Samen erzeugen, die den Anstieg im Ölgehalt zeigen.

**11.** Verfahren zur Herstellung einer Pflanze, umfassend:

(a) das Transformieren einer Pflanze mit einem Nukleinsäurekonstrukt, umfassend eine Nukleinsäure, welche eine cytosolische ACCase kodiert, die funktionell mit einem Promoter verbunden ist, wobei dem Konstrukt eine Nukleinsäure fehlt, die ein Transitpeptid kodiert, welches funktionell mit der die cytosolische ACCase kodierenden Nukleinsäure verbunden ist, und
(b) das Auswählen von Nachkommen-Pflanzen, nach mindestens einer Generation ohne Auswahl, die Samen erzeugen, welche einen relativen Anstieg im Ölgehalt verglichen mit Samen zeigen, die von einer entsprechenden Pflanze erzeugt werden, der das Nukleinsäurekonstrukt fehlt, wobei der relative Anstieg im Ölgehalt von 5% bis 25% auf Basis des Trockengewichts beträgt.

**12.** Verfahren nach Anspruch 11, wobei die Nukleinsäure eine cytosolische Pflanzen-ACCase kodiert.

**13.** Verfahren nach Anspruch 12, wobei die Nukleinsäure eine cytosolische Alfalfa-ACCase kodiert.

**14.** Verfahren nach Anspruch 11, wobei das Auswählen für mindestens drei Generationen ist.

**15.** Verfahren nach Anspruch 11, wobei der Promoter ein CaMV 35S-Promoter ist.

**16.** Verfahren nach Anspruch 11, wobei der die cytosolische ACCase kodierenden Nukleinsäure Introns fehlen.

**17.** Verfahren nach Anspruch 11, wobei die Pflanze eine Brassica-Pflanze ist.

**18.** Verfahren nach Anspruch 11, wobei die Pflanze aus der Gruppe, bestehend aus *Brassica napus, Brassica rapa*, *Brassica juncea, Brassica carinata, Brassica nigra* und *Brassica oleracea* ausgewählt ist.

**19.** Verfahren zur Herstellung einer Pflanze, umfassend die Schritte:

(a) des Einfügens eines Konstrukts in eine oder mehrere Pflanzen, wobei das Konstrukt eine Nukleinsäure umfasst, welche eine cytosolische Acetyl-ACCase kodiert, die funktionell mit einem Promoter verbunden ist, wobei dem Konstrukt eine Nukleinsäure fehlt, die ein Transitpeptid kodiert, welches funktionell mit der die cytosolische ACCase kodierenden Nukleinsäure verbunden ist, wobei

die Nachkommen einer oder mehrerer der transgenen Pflanzen, nachfolgend mindestens einer Generation ohne Auswahl, Samen erzeugen, welche einen relativen Anstieg im Ölgehalt verglichen mit Samen zeigen, die von einer entsprechenden Pflanze erzeugt werden, der das Nukleinsäurekonstrukt fehlt, welches die ACCase kodiert, wobei der relative Anstieg im Ölgehalt von 5% bis 25% auf Basis des Trockengewichts beträgt.

**20.** Verfahren zur Steigerung des Ölgehalts in Samen, umfassend die Schritte:

(a) des Erzeugens einer oder mehrerer transgener T1-Pflanzen, welche ein rekombinantes Nukleinsäurekonstrukt enthalten und exprimieren, wobei das rekombinante Nukleinsäurekonstrukt eine Nukleinsäure umfasst, welche eine cytosolische ACCase kodiert, die funktionell mit einem Promoter verbunden ist, wobei dem Konstrukt eine Nukleinsäure fehlt, die ein Transitpeptid kodiert, welches funktionell mit der die cytosolische ACCase kodierenden Nukleinsäure verbunden ist, und
(b) des Auswählens von T3- oder späteren Nachkommen der einen oder mehreren Pflanzen, welche einen relativen Anstieg im Samenölgehalt verglichen mit Samen zeigen, die von einer entsprechenden Pflanze erzeugt werden, der das Nukleinsäurekonstrukt fehlt, wobei der relative Anstieg im Ölgehalt von 5% bis 25% auf Basis des Trockengewichts beträgt.

**21.** Verfahren nach Anspruch 20, wobei der Auswahl-Schritt das Auswählen von Nachkommen umfasst, welche das Nukleinsäurekonstrukt enthalten.

**Revendications**

**1.** Plante transgénique qui contient et exprime un construct d'acide nucléique recombinant, ledit construct d'acide nucléique recombinant comprenant un acide nucléique codant pour une acétyl coenzyme A carboxylase (ACCase) cytosolique liée de façon à coopérer avec un promoteur, ledit construct étant dépourvu d'acide nucléique codant pour un peptide de transit lié de façon à coopérer avec ledit acide nucléique codant pour ladite ACCase cytosolique, où ladite plante transgénique produit des grains qui présentent une teneur en huile relativement augmentée par comparaison à des grains produits par une plante correspondante dépourvue dudit construct d'acide nucléique, ladite augmentation relative de la teneur en huile étant comprise entre 5 % et 25 % sur la base du poids à sec.

**2.** Plante selon la revendication 1, dans laquelle ledit acide nucléique code pour une ACCase cytosolique végétale.

**3.** Plante selon la revendication 2, dans laquelle ledit acide nucléique code pour une ACCase cytosolique de luzerne.

**4.** Plante selon la revendication 1, dans laquelle ledit promoteur est un promoteur 35S du virus de la mosaïque du chou-fleur (CaMV).

**5.** Plante selon la revendication 1 ou 4, dans laquelle ledit acide nucléique codant pour ladite ACCase cytosolique est dépourvu d'introns.

**6.** Plante selon la revendication 1, ladite plante étant une plante de soja.

**7.** Plante selon la revendication 1, ladite plante étant un chou *(Brassica).*

**8.** Plante selon la revendication 7, ladite plante étant choisie dans le groupe constitué de *Brassica napus, Brassica rapa, Brassica juncea, Brassica carinata, Brassica nigra* et *Brassica oleracea.*

**9.** Grains produits par la plante selon la revendication 1, lesdits grains comprenant ledit construct d'acide nucléique recombinant.

**10.** Descendance de la plante selon la revendication 1, dans laquelle lesdits grains descendants comprennent et expriment ledit construct d'acide nucléique recombinant, ladite descendance produisant des grains qui font apparaître ladite augmentation de la teneur en huile.

**11.** Procédé d'obtention d'une plante, comprenant les étapes consistant à :

a) transformer une plante avec un construct d'acide nucléique comprenant un acide nucléique codant pour une ACCase cytosolique liée de façon à coopérer avec un promoteur, ledit construct étant dépourvu d'acide nucléique codant pour un peptide de transit lié de façon à coopérer avec ledit acide nucléique codant pour ladite ACCase cytosolique ; et
b) sélectionner, après au moins une génération sans sélection, des plantes descendantes qui produisent des grains faisant apparaître une augmentation relative de la teneur en huile dans le grain par comparaison à des grains produits par une plante correspondante dépourvue dudit construct d'acide nucléique, ladite augmentation relative de la teneur en huile étant comprise entre 5 et 25 % sur la base du poids sec.

**12.** Procédé selon la revendication 11, dans lequel ledit acide nucléique code pour une ACCase cytosolique végétale.

**13.** Procédé selon la revendication 12, dans lequel ledit acide nucléique code pour une ACCase cytosolique de luzerne.

**14.** Procédé selon la revendication 11, dans lequel ladite sélection porte sur au moins trois générations.

**15.** Procédé selon la revendication 11, dans lequel ledit promoteur est un promoteur 35S du virus CaMV.

**16.** Procédé selon la revendication 11, dans lequel ledit acide nucléique codant pour ladite ACCase cytosolique est dépourvu d'introns.

**17.** Procédé selon la revendication 11, dans lequel ladite plante est un chou *(Brassica).*

**18.** Procédé selon la revendication 11, dans lequel ladite plante est choisie dans le groupe constitué de *Brassica napus, Brassica rapa, Brassica juncea, Brassica carinata, Brassica nigra* et *Brassica oleracea.*

**19.** Procédé d'obtention d'une plante, comprenant les étapes consistant à :

a) introduire un construct dans une ou plusieurs plante(s), ledit construct comprenant un acide nucléique codant pour une ACCase cytosolique liée de façon à coopérer avec un promoteur, ledit construct étant dépourvu d'acide nucléique codant pour un peptide de transit lié de façon à coopérer avec ledit acide nucléique codant pour ladite ACCase cytosolique ;

dans lequel la descendance d'une ou de plusieurs desdites plantes transgéniques, après au moins une génération sans sélection, produit des grains faisant apparaître une augmentation relative de la teneur en huile par comparaison à des grains produits par une plante correspondante dépourvue dudit acide nucléique codant pour ladite ACCase, ladite augmentation relative de la teneur en huile étant comprise entre 5 et 25 % sur la base du poids sec.

**20.** Procédé d'augmentation de la teneur en huile dans des grains, comprenant les étapes consistant à :

a) créer une ou plusieurs plante(s) transgénique(s) T1 contenant et exprimant un construct d'acide nucléique recombinant, ledit construct d'acide nucléique recombinant comprenant un acide nucléique codant pour une ACCase cytosolique liée de façon à coopérer avec un promoteur ; ledit construct étant dépourvu d'acide nucléique codant pour un peptide de transit lié de façon à coopérer avec ledit acide nucléique codant pour ladite ACCase cytosolique ; et
b) sélectionner une descendance T3 ou ultérieure de ladite ou desdites plante(s) faisant apparaître une augmentation relative de la teneur en huile des grains par comparaison à des grains produits par une plante correspondante dépourvue dudit construct d'acide nucléique, ladite augmentation relative de la teneur en huile étant comprise entre 5 et 25 % sur la base du poids sec.

**21.** Procédé selon la revendication 20, dans lequel ladite étape de sélection comprend la sélection de la descendance qui contient ledit construct d'acide nucléique.

atggcttcctcagttctttcctctgcagcagttgccacccgcagcaatgttgctcaagct
M A S S V L S S A A V A T R S N V A Q A

aacatggttgcacctttcactggccttaagtcagctgcctcattccctgtttcaaggaag
N M V A P F T G L K S A A S F P V S R K

caaaaccttgacatcacttccattgccagcaacggcggaagagtgcaatgcatgcaggtg
Q N L D I T S I A S N G G R V Q C M Q V

tggccaccaattaacaagaagtcc (SEQ ID NO:3)
W P P I N K K S (SEQ ID NO:4)

*Figure 1*

M

ssRubisco
transit

(CaMV 35S)ggtaccaaaaaaaacaaccATGgcttcctcagttctttcctctgcagcagttgccacccgcagcaatgttgctcaag

KpnI

ctaacatggttgcacctttcactggccttaagtcagctgcctcattccctgtttcaaggaagcaaaaccttgacatcacttccatt

gccagcaacggcggaagagtgcaatgcATGCAGGTGTGGCCACCAATTAACAAGAAGTCC**ATG**--------Ccttttataaaa

ssRubisco
mature

Alfalfa
ACCase

3' ACCase
sequences

aaaaaaaaaagaattcctgcagcccggggatcc (SEQ ID NO:5)

pAMP1 vector
sequences

BamHI

**Figure 2**

M ↓ Alfalfa ACCase ↓ 3' ACCase sequences ↓ pAMP1 vector sequences

(CaMV 35S)ggtaccaaaaaaacaaccATG----------Ccttttataaaaaaaaaag aattcctgcagcccggggatcc(SEQ ID NO:6)

KpnI (under ggtacc) BamHI (under ggatcc)

*Figure 3*

```
atggctagcgtgggccgtggaaatggatatttaaacagtgtgctaccgagtaggcaccctgctactacaaccgaa
  M  A  S  V  G  R  G  N  G  Y  L  N  S  V  L  P  S  R  H  P  A  T  T  T  E

gtagatgaatactgcaatgcccttggaggaaacaagccgattcatagcatattgattgcaaacaatggaatggca
  V  D  E  Y  C  N  A  L  G  G  N  K  P  I  H  S  I  L  I  A  N  N  G  M  A

gcagtcaagtttatacgtagtgttaggagttgggcttacgagacatttggcacggaaaaagctatcttgttggtt
  A  V  K  F  I  R  S  V  R  S  W  A  Y  E  T  F  G  T  E  K  A  I  L  L  V

gccatggcaactccagaggatatgagaatcaatgcagaacatatcagaatagccgatcaatttgtggaagtacct
  A  M  A  T  P  E  D  M  R  I  N  A  E  H  I  R  I  A  D  Q  F  V  E  V  P

ggtgggaccaataacaataactacgccaatgtgcagcttattctagagattgctgagataactcacgttgatgcg
  G  G  T  N  N  N  N  Y  A  N  V  Q  L  I  L  E  I  A  E  I  T  H  V  D  A

gtgtggcctggttggggtcatgcatcagaaaatcctgagcttccagatgcattaaaagcaaagggaattgtattc
  V  W  P  G  W  G  H  A  S  E  N  P  E  L  P  D  A  L  K  A  K  G  I  V  F

cttggacctcctgctatatctatggcagcattgggagacaaaattggttcctcgttgattgctcaggcagcagaa
  L  G  P  P  A  I  S  M  A  A  L  G  D  K  I  G  S  S  L  I  A  Q  A  A  E

gttccaacccttccatggagtggttctcatgtgaaaattcctccagaaagtgacttgattactattcctgatgaa
  V  P  T  L  P  W  S  G  S  H  V  K  I  P  P  E  S  D  L  I  T  I  P  D  E

atttaccgtgcagcatgtgtttatacaacagaagaagcaattgcaagttgtcaagtagtaggttaccctgcaatg
  I  Y  R  A  A  C  V  Y  T  T  E  E  A  I  A  S  C  Q  V  V  G  Y  P  A  M

attaaggcatcttggggtggtggcggcaaaggcataagaaaggttcataatgatgatgaggttagggcattgttc
  I  K  A  S  W  G  G  G  G  K  G  I  R  K  V  H  N  D  D  E  V  R  A  L  F

aagcaagttcaaggtgaagtaccaggctcacctatatttataatgaaagttgcttcccagagccgacatcttgaa
  K  Q  V  Q  G  E  V  P  G  S  P  I  F  I  M  K  V  A  S  Q  S  R  H  L  E

gtccaattgatttgcgatcagcacggaaattttgcagcattgcacagccgtgattgtagtgttcaaagaaggcat
  V  Q  L  I  C  D  Q  H  G  N  F  A  A  L  H  S  R  D  C  S  V  Q  R  R  H

caaaagattattgaagagggtcccattactgtagcacctccagaaacggtgaaagaacttgaacaggcggctaga
  Q  K  I  I  E  E  G  P  I  T  V  A  P  P  E  T  V  K  E  L  E  Q  A  A  R

agattagctaaatctgtaaattatgtgggggcagctaccgttgagtatctttatagcatggaaactggcgagtac
  R  L  A  K  S  V  N  Y  V  G  A  A  T  V  E  Y  L  Y  S  M  E  T  G  E  Y

tactttttagagttgaacccccgactacaggttgagcatcctgttactgaatggatagctgagataaatctgcca
  Y  F  L  E  L  N  P  R  L  Q  V  E  H  P  V  T  E  W  I  A  E  I  N  L  P

gcagcacaagttgcagttgggatgggcatcccactctggcaaattcctgagattaggcgtttctatgggatggaa
  A  A  Q  V  A  V  G  M  G  I  P  L  W  Q  I  P  E  I  R  R  F  Y  G  M  E

catggtggggaaatgatggttggaagaaaacatcagtgttagctacccctttgattttgacgaagcacaatct
  H  G  G  G  N  D  G  W  K  K  T  S  V  L  A  T  P  F  D  F  D  E  A  Q  S
```

***Figure 4-1***

```
acaaagccgaaaggtcattgtgtggctgtacgagtcaccagtgaggaccccgatgatggttttacgcctacagga
  T  K  P  K  G  H  C  V  A  V  R  V  T  S  E  D  P  D  D  G  F  T  P  T  G

ggaaaagtgcaggagctcagctttaaaagcaagccaaatgtgtgggcttatttctctgttaagtccggaggagga
  G  K  V  Q  E  L  S  F  K  S  K  P  N  V  W  A  Y  F  S  V  K  S  G  G  G

attcatgaattctcagattctcaatttggacatgtttttgcgtttggagaatctagagctttagcaattgcaaat
  I  H  E  F  S  D  S  Q  F  G  H  V  F  A  F  G  E  S  R  A  L  A  I  A  N

atggtactggggttgaaggaaattcaaattcgaggagaaattcgtaccaacgttgattacacaattgatcttctg
  M  V  L  G  L  K  E  I  Q  I  R  G  E  I  R  T  N  V  D  Y  T  I  D  L  L

aatgcttcagactacagagacaacaaaattcacacaggatggctagacagtagaattgcaatgcgggttagagca
  N  A  S  D  Y  R  D  N  K  I  H  T  G  W  L  D  S  R  I  A  M  R  V  R  A

gagaggcctccctggtatctgtctgttgttggtggggcactctataaagcttctgccagcagtgcagctttagtt
  E  R  P  P  W  Y  L  S  V  V  G  G  A  L  Y  K  A  S  A  S  S  A  A  L  V

tcggactatgttggctatcttgaaaaggggcaaatccctcccaagcacatttctcttgtccattctcaagtttct
  S  D  Y  V  G  Y  L  E  K  G  Q  I  P  P  K  H  I  S  L  V  H  S  Q  V  S

ttgagcattgaaggaagcaaatacacgattgacatggtacgaggaggacctggaagttacaaattgaaattgaat
  L  S  I  E  G  S  K  Y  T  I  D  M  V  R  G  G  P  G  S  Y  K  L  K  L  N

caatcggagatagaagcggagatacacactttacgtgatggaggtttgctaatgcagttggatggaaacagtcat
  Q  S  E  I  E  A  E  I  H  T  L  R  D  G  G  L  L  M  Q  L  D  G  N  S  H

gtaatatatgcagaggaagaagcagctggaactcggcttttaatagatggaaggacttgcttgcttcagaatgat
  V  I  Y  A  E  E  E  A  A  G  T  R  L  L  I  D  G  R  T  C  L  L  Q  N  D

gacgatccatcaaagttaattggagagacaccgtgcaagcttctgagatatttggttgcggatgatagtcagatt
  D  D  P  S  K  L  I  G  E  T  P  C  K  L  L  R  Y  L  V  A  D  D  S  Q  I

gatgcagacacaccatatgctgaagttgaggtcatgaagatgtgcatgcctcttctttcccctgcttctggaatt
  D  A  D  T  P  Y  A  E  V  E  V  M  K  M  C  M  P  L  L  S  P  A  S  G  I

attcatttcagaatggctgaaggtcaagccatgcaggctggtgaacttatagcaaagcttgatctagatgatggt
  I  H  F  R  M  A  E  G  Q  A  M  Q  A  G  E  L  I  A  K  L  D  L  D  D  G

tctgcagtaaggaaggcagaacccttcactgggagcttccctatcctgggccctcctactgcaatttcaggtaaa
  S  A  V  R  K  A  E  P  F  T  G  S  F  P  I  L  G  P  P  T  A  I  S  G  K

gttcatcagaaatgtgcagcaagcttaaacgctgcacggatgattcttgctggctatgagcacaacattgatgaa
  V  H  Q  K  C  A  A  S  L  N  A  A  R  M  I  L  A  G  Y  E  H  N  I  D  E

gttgtggtcaaaagtttgctcaattgccttgacagccctgaactgcctttccttcaatggcaagagtgctttgca
  V  V  V  K  S  L  L  N  C  L  D  S  P  E  L  P  F  L  Q  W  Q  E  C  F  A

gttttggcaacccgtcttcccaaagatcttagaaacgagttggaagctaaatataaggagttcgaaattatttca
  V  L  A  T  R  L  P  K  D  L  R  N  E  L  E  A  K  Y  K  E  F  E  I  I  S
```

***Figure 4-2***

agctcccaaactattgatttccctgccaaattattgaaggcaatccttgaagctcatctttcctcctgtcctgaa
S S Q T I D F P A K L L K A I L E A H L S S C P E

aacgaaaaaggagccttagaaagactagttgaaccgctgacaagtcttgtaaagtcttatgagggtggaagagag
N E K G A L E R L V E P L T S L V K S Y E G G R E

agccatgctcataaaattgttcaatctctatttgaagagtatctttcagttgaagaactattcagtgataatata
S H A H K I V Q S L F E E Y L S V E E L F S D N I

caggctgatgtaattgaacgactccgtcttcaatacaagaaagatttgttgaagattgtagatattgtgctctct
Q A D V I E R L R L Q Y K K D L L K I V D I V L S

catcagggtgtcaagagcaaaaacaagctgatactgcgactaatggataaactggtttaccctaatcctgctgcc
H Q G V K S K N K L I L R L M D K L V Y P N P A A

tatagggatcaattaatccgattctcccaactcaaccatatagtttattctgagttggctcttaaggcaagtcaa
Y R D Q L I R F S Q L N H I V Y S E L A L K A S Q

ctgttggagcaaactaaactcagtgaacttcgatccagcattgctagaagtctttctgaactagaaatgtttacc
L L E Q T K L S E L R S S I A R S L S E L E M F T

gaggatggtgaaaatattgatactccgaagaggaagagtgccattaatgacagaatggaggaccttgtgagcgct
E D G E N I D T P K R K S A I N D R M E D L V S A

cctttggctgttgaagatgcccttgttggtttatttgatcacagcgatcacacccttcaaaggagagttgttgaa
P L A V E D A L V G L F D H S D H T L Q R R V V E

acttatatccgtaggctctatcagccatatcttgtcaaagatagcatcaggatgcagtggcacagatctggcctt
T Y I R R L Y Q P Y L V K D S I R M Q W H R S G L

attgctacatgggaattcttagaagaatacgttgaacggaagaatggggttgaagacaaaacactggtggagaaa
I A T W E F L E E Y V E R K N G V E D K T L V E K

catagtgagaagaaatggggagtgatggttgtaattaaatctcttcagtttttgccagcaattatcagtgctgca
H S E K K W G V M V V I K S L Q F L P A I I S A A

ttaagagaagcaaccaataactttcacgatcctcttaaaagtggttctggtgactcaagtaaccatggtaatatg
L R E A T N N F H D P L K S G S G D S S N H G N M

atgcatattggattagtggggatcaacaaccaaatgagtttacttcaagacagtggtgatgaggatcaggctcaa
M H I G L V G I N N Q M S L L Q D S G D E D Q A Q

gaaagaattgataagttggccaaaatactcagagagcaggaaatagggtccataatacatgctgcaggtgttgga
E R I D K L A K I L R E Q E I G S I I H A A G V G

gatattagctgtatcatacagagggatgaagggcgtgctccaatgaggcattcctttcactggtcatctgaaaag
D I S C I I Q R D E G R A P M R H S F H W S S E K

ctatattatgtagaggaaccattgttgctccatcttgaacctcccctatccatttatcttgaactggacaagctt
L Y Y V E E P L L L H L E P P L S I Y L E L D K L

***Figure 4-3***

```
aagtgctatgaaaatattcgctatacaccatcccgagatcgtcaatggcacctctacacagttgtggataccaag
 K  C  Y  E  N  I  R  Y  T  P  S  R  D  R  Q  W  H  L  Y  T  V  V  D  T  K

ccacaaccaattcaaagaatgtttcttcgaacacttatcagacagccaaccacaaatgaaggatactcttcttat
 P  Q  P  I  Q  R  M  F  L  R  T  L  I  R  Q  P  T  T  N  E  G  Y  S  S  Y

caaagactggatgcagaaacgtcccgtacccaattggctatgtcttatacttcaaggagcatttttaggtcccta
 Q  R  L  D  A  E  T  S  R  T  Q  L  A  M  S  Y  T  S  R  S  I  F  R  S  L

atgggcgcaatggaggagttggaacttaactcacacaataccaccatcaaatctgaacatgctcatatgtacctc
 M  G  A  M  E  E  L  E  L  N  S  H  N  T  T  I  K  S  E  H  A  H  M  Y  L

tatatcatacgcgagcagcaaatagatgatcttgtgccttattccaagaaaattaacatagaagctggccaagaa
 Y  I  I  R  E  Q  Q  I  D  D  L  V  P  Y  S  K  K  I  N  I  E  A  G  Q  E

gaaacaacagttgaggcaatcttggaagaactggcacaggaaatccattcctctgttggtgtaagaatgcacaga
 E  T  T  V  E  A  I  L  E  E  L  A  Q  E  I  H  S  S  V  G  V  R  M  H  R

ttaggcgttttcgtgtgggaaatcaagctctggattacagcatgtggacaggcaaatggtgcttggagggtcatt
 L  G  V  F  V  W  E  I  K  L  W  I  T  A  C  G  Q  A  N  G  A  W  R  V  I

gtaaacaatgtgactggtcatacatgcactgtacatatatatcgagagatggaggatgccaccactcataaagtg
 V  N  N  V  T  G  H  T  C  T  V  H  I  Y  R  E  M  E  D  A  T  T  H  K  V

gtctacagttcagtcactgtaaagggtccgttgcatggtgtaccggtgaatgaaaactatcaacctttgggaggt
 V  Y  S  S  V  T  V  K  G  P  L  H  G  V  P  V  N  E  N  Y  Q  P  L  G  G

attgaccgaaaacgtcttgcagcgagaaagaacagcaccacatactgctatgatttccccttgcatttcaaaca
 I  D  R  K  R  L  A  A  R  K  N  S  T  T  Y  C  Y  D  F  P  L  A  F  Q  T

tccttggaacagtcctggtcaatacagcagacaggaattcaaagagctaatgataaggatctcctaaaagtaaca
 S  L  E  Q  S  W  S  I  Q  Q  T  G  I  Q  R  A  N  D  K  D  L  L  K  V  T

gagcttaaattttccgaaaaagctggtagttggggtacttctcttgttcctgcagagcgtcttcctggactcaat
 E  L  K  F  S  E  K  A  G  S  W  G  T  S  L  V  P  A  E  R  L  P  G  L  N

gatgttggcatggtagcctggttgatggaaatgtgtacgcctaaattcccatctggaaggacaatattggttgtt
 D  V  G  M  V  A  W  L  M  E  M  C  T  P  K  F  P  S  G  R  T  I  L  V  V

tcaaacgatgtgaccttcaaggccgggtcttttggcccaagagaggatgcattctttagagcagtaactgatctt
 S  N  D  V  T  F  K  A  G  S  F  G  P  R  E  D  A  F  F  R  A  V  T  D  L

gcctgtgcaaagaaaatacctttaatttacttggcagcaaattctggtgcccgtttaggtgttgccgaggaagtc
 A  C  A  K  K  I  P  L  I  Y  L  A  A  N  S  G  A  R  L  G  V  A  E  E  V

aaagcttgtttcaaagttggttggtctgaggaatctaaacctgaacatggttttcagtatgtatatttaacacct
 K  A  C  F  K  V  G  W  S  E  E  S  K  P  E  H  G  F  Q  Y  V  Y  L  T  P

gaggattatgctcgaatcggatcatcagtgatggcacatgaattaaagcttgaaagtggagaaaccagatgggtt
 E  D  Y  A  R  I  G  S  S  V  M  A  H  E  L  K  L  E  S  G  E  T  R  W  V
```

***Figure 4-4***

```
atagataccattgttggcaaagaagatggactgggagttgaaaacttgagtggtagtggggccattgccggtgcc
  I  D  T  I  V  G  K  E  D  G  L  G  V  E  N  L  S  G  S  G  A  I  A  G  A

tattcaagggcatacaaggaaacctttacattgacatatgttaccggtaggactgttggaattggtgcttatctt
  Y  S  R  A  Y  K  E  T  F  T  L  T  Y  V  T  G  R  T  V  G  I  G  A  Y  L

gctaggcttgggatgaggtgcatacagaggcttgatcaacctataattcttaccgggttttcagcattaaacaaa
  A  R  L  G  M  R  C  I  Q  R  L  D  Q  P  I  I  L  T  G  F  S  A  L  N  K

cttcttggtagggaggtgtacagctctcacatgcaacttggtggaccgaaaatcatggcaacaaatggagtcgtt
  L  L  G  R  E  V  Y  S  S  H  M  Q  L  G  G  P  K  I  M  A  T  N  G  V  V

catctcacagtttcggacgaccttgaaggcgtttcttctattttgaagtggcttagctacgttccttctcatgta
  H  L  T  V  S  D  D  L  E  G  V  S  S  I  L  K  W  L  S  Y  V  P  S  H  V

ggtggtgcacttcccattgtaaagccccttgatcccccagagagggaagtggagtatttaccggaaaattcatgc
  G  G  A  L  P  I  V  K  P  L  D  P  P  E  R  E  V  E  Y  L  P  E  N  S  C

gatcctcgtgctgccatttccggaactctggatgttaatggaaagtggctgggaggcattttgacaaggacagc
  D  P  R  A  A  I  S  G  T  L  D  V  N  G  K  W  L  G  G  I  F  D  K  D  S

tttgtggagacactagaaggatgggctagaacagttgttacaggaagggcaaagcttggaggaatccctgtggga
  F  V  E  T  L  E  G  W  A  R  T  V  V  T  G  R  A  K  L  G  G  I  P  V  G

attgttgcggtggaaacacaaacagttatgcaaataatacctgctgatccaggtcaacttgattctcacgagagg
  I  V  A  V  E  T  Q  T  V  M  Q  I  I  P  A  D  P  G  Q  L  D  S  H  E  R

gttgttcctcaagccgggcaggtgtggtttcctgattctgcgaccaagacggcccaagcgatattggatttcaac
  V  V  P  Q  A  G  Q  V  W  F  P  D  S  A  T  K  T  A  Q  A  I  L  D  F  N

agagaagaactcccactttcattatcgcaaactggagaggcttttcaggtggacaaaggaccttttgaagga
  R  E  E  L  P  L  F  I  I  A  N  W  R  G  F  S  G  G  Q  R  D  L  F  E  G

attcttcaggctggttcgactattgtggagaaccttaggacatacaaacagcccatatttgtatacattccaatg
  I  L  Q  A  G  S  T  I  V  E  N  L  R  T  Y  K  Q  P  I  F  V  Y  I  P  M

atgggtgaactccgaggcggggcttgggttgttgtcgacagccgaatcaactcagaccacattgaaatgtatgct
  M  G  E  L  R  G  G  A  W  V  V  V  D  S  R  I  N  S  D  H  I  E  M  Y  A

gagcgaacggccaaaggtaacgtccttgagccggaaggaatgattgaaatcaaatttagaacaagagaattgttg
  E  R  T  A  K  G  N  V  L  E  P  E  G  M  I  E  I  K  F  R  T  R  E  L  L

gagtgtatgagaagacttgatcaacaattgattaatttgaaggaaaaactttctgaagccaagagtaacaaggac
  E  C  M  R  R  L  D  Q  Q  L  I  N  L  K  E  K  L  S  E  A  K  S  N  K  D

tatggtgcatatgattctctgcagcagcagattagattccgtgagaaacagcttttgcctttgtatactcagata
  Y  G  A  Y  D  S  L  Q  Q  Q  I  R  F  R  E  K  Q  L  L  P  L  Y  T  Q  I

gctacaaaatttgctgaactccatgatacttcattaagaatgaaagcaaagggtgtaatcagagaagttcttgat
  A  T  K  F  A  E  L  H  D  T  S  L  R  M  K  A  K  G  V  I  R  E  V  L  D
```

## *Figure 4-5*

```
tggcgtaagtcgcgttctgtcttctatcagagactgcacaggagaatcggtgagcactcactgatcaacatcgtg
  W  R  K  S  R  S  V  F  Y  Q  R  L  H  R  R  I  G  E  H  S  L  I  N  I  V

agagatgctgctggtgaccaattgtcatatgtttctgccatgaacttgctcaaagaatggtatctgaattctgat
  R  D  A  A  G  D  Q  L  S  Y  V  S  A  M  N  L  L  K  E  W  Y  L  N  S  D

atcgccaaaggtagagaagatgcttggttggacgatgaagccttcttcagatggagggatgatccagcaaactac
  I  A  K  G  R  E  D  A  W  L  D  D  E  A  F  F  R  W  R  D  D  P  A  N  Y

gaggataaactaaaggaattgcgcgtccagagactgttgcttcagttgacaaatattggcgactcggctctagat
  E  D  K  L  K  E  L  R  V  Q  R  L  L  L  Q  L  T  N  I  G  D  S  A  L  D

ttacaagctctacctcaaggtcttgccgcccttttaagcaagttggaagcatcaagtcgcgataagttgatcagt
  L  Q  A  L  P  Q  G  L  A  A  L  L  S  K  L  E  A  S  S  R  D  K  L  I  S

gaacttcgcaaagtactcggttagtagacagtgaatgctcctgtgatctgcccatgcactcatgttgtagtgttc
  E  L  R  K  V  L  G          (SEQ ID NO:8)

acgtcgttgatacatgaccatatagaaatgtatccattttacgatgttatcatcaaagtagcagcatccctcgga

aaatggactttcacttgagggatcaactgtaaatgacttcggtcttggatagatatttaatttatgcagttagag

gatcataaccagcatcaccatgtttggtctatttatttgctggttgattgattctttgcgtgtatctgaataaac

atgtaataatttgtaacattgattatttttatgaaaaacaaagttttgggcactccttttataaaaaaaaaaaa

aagaattcctgcagcccgggggatcc       (SEQ ID NO:7)
```

*Figure 4-6*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5925805 A
- WO 0007430 A
- US 5204253 A
- US 6051756 A
- US 5188958 A
- US 5484956 A
- US 5550318 A
- US 5969169 A
- US 5850026 A
- US 60198794 B


### Non-patent literature cited in the description


- **WHITE et al.** Adv. in Plant Lipid Res. Universidad De Sevilla, Spain, 1998, 62-66
- **ROESLER et al.** *Plant Physiol.,* 1997, vol. 113, 75-81
- **SAMBROOK et al.** Molecular Cloning: A Laboratory Manual. Cold Spring Harbor Laboratory Press, 1989
- **WEISING et al.** *Ann. Rev. Genetics,* 1988, vol. 22, 421-478
- **DAYHOFF et al.** *Atlas of Protein Sequence and Structure,* 1978, vol. 5, 345-352
- **WALKERPEACH et al.** Plant Molecular Biology Manual, Gelvin & Schilperoort. 1994, vol. 1, 1-19
- Castor: Return of an old crop. **BRIGHAM RD.** New Crops. Wiley, 1993, 380-3
- Edible Oil & Fat Products: Oils and Oil Seeds. Bailey's Industrial Oil & Fat Products. Wiley, 1996, vol. 2
- **SHORROSH et al.** *Proc. Natl. Acad. Sci. USA,* 1994, vol. 91, 4323-27
- Principles in Cultivar Development. Crop Species, vol. 2, 443
- **MCVETTY et al.** *Can. J. Plant Sci.,* 1996, vol. 76 (2), 341-342
- **SCARTH et al.** *Can. J. Plant Sci.,* 1995, vol. 75 (1), 205-206
- **MC VETTY et al.** *Can J. Plant Sci.,* 1996, vol. 76 (2), 343-344

SEQUENCE LISTING

<110> Cargill Incorporated

<120> PLANTS CONTAINING A CYTOSOLIC ACETYL COA-CARBOXYLASE

<130> 07148-094WO1

<150> US 60/198,794
<151> 2000-04-20

<160> 12

<170> FastSEQ for Windows Version 4.0

<210> 1

<211> 56
<212> DNA
<213> Artificial Sequence

<220>
<223> primer for PCR

<400> 1
caucaucauc auatcgatag gtaccaaaaa aaacaaccat ggcttcctca gttctt 56

<210> 2
<211> 45
<212> DNA
<213> Artificial Sequence

<220>
<223> primer for PCR

<400> 2
cuacuacuac uagctagcca tggacttctt gttaattggt ggcca 45

<210> 3
<211> 204
<212> DNA
<213> Nicotiana tabacum

<220>
<221> CDS
<222> (1) ... (204)

<400> 3

```
atg gct tcc tca gtt ctt tcc tct gca gca gtt gcc acc cgc agc aat       48
Met Ala Ser Ser Val Leu Ser Ser Ala Ala Val Ala Thr Arg Ser Asn
 1               5                  10                  15

gtt gct caa gct aac atg gtt gca cct ttc act ggc ctt aag tca gct       96
Val Ala Gln Ala Asn Met Val Ala Pro Phe Thr Gly Leu Lys Ser Ala
            20                  25                  30

gcc tca ttc cct gtt tca agg aag caa aac ctt gac atc act tcc att      144
Ala Ser Phe Pro Val Ser Arg Lys Gln Asn Leu Asp Ile Thr Ser Ile
        35                  40                  45

gcc agc aac ggc gga aga gtg caa tgc atg cag gtg tgg cca cca att      192
Ala Ser Asn Gly Gly Arg Val Gln Cys Met Gln Val Trp Pro Pro Ile
    50                  55                  60

aac aag aag tcc                                                      204
Asn Lys Lys Ser
 65
```

<210> 4
<211> 68
<212> PRT

<213> Nicotiana tabacum

<400> 4

```
Met Ala Ser Ser Val Leu Ser Ser Ala Ala Val Ala Thr Arg Ser Asn
 1               5                  10                  15
Val Ala Gln Ala Asn Met Val Ala Pro Phe Thr Gly Leu Lys Ser Ala
            20                  25                  30
Ala Ser Phe Pro Val Ser Arg Lys Gln Asn Leu Asp Ile Thr Ser Ile
        35                  40                  45
Ala Ser Asn Gly Gly Arg Val Gln Cys Met Gln Val Trp Pro Pro Ile
    50                  55                  60
Asn Lys Lys Ser
65
```

<210> 5
<211> 149
<212> DNA
<213> Artificial Sequence

<220>
<223> representative construct (5' end)

<400> 5

```
ctaacatggt tgcacctttc actggcctta agtcagctgc ctcattccct gtttcaagga    60
agcaaaacct tgacatcact tccattgcca gcaacggcgg aagagtgcaa tgcatgcagg   120
tgtggccacc aattaacaag aagtccatg                                     149
```

<210> 6
<211> 22
<212> DNA
<213> Artificial Sequence

<220>
<223> representative construct (5' end)

<400> 6
ggtaccaaaa aaaacaacca tg 22

<210> 7
<211> 7151
<212> DNA
<213> Medicago sativa

<220>
<221> CDS
<222> (1)...(6771)

<400> 7

```
atg gct agc gtg ggc cgt gga aat gga tat tta aac agt gtg cta ccg     48
```

```
Met Ala Ser Val Gly Arg Gly Asn Gly Tyr Leu Asn Ser Val Leu Pro
 1               5                  10                  15

agt agg cac cct gct act aca acc gaa gta gat gaa tac tgc aat gcc      96
Ser Arg His Pro Ala Thr Thr Thr Glu Val Asp Glu Tyr Cys Asn Ala
            20                  25                  30

ctt gga gga aac aag ccg att cat agc ata ttg att gca aac aat gga     144
Leu Gly Gly Asn Lys Pro Ile His Ser Ile Leu Ile Ala Asn Asn Gly
        35                  40                  45

atg gca gca gtc aag ttt ata cgt agt gtt agg agt tgg gct tac gag     192
Met Ala Ala Val Lys Phe Ile Arg Ser Val Arg Ser Trp Ala Tyr Glu
    50                  55                  60

aca ttt ggc acg gaa aaa gct atc ttg ttg gtt gcc atg gca act cca     240
Thr Phe Gly Thr Glu Lys Ala Ile Leu Leu Val Ala Met Ala Thr Pro
65                  70                  75                  80

gag gat atg aga atc aat gca gaa cat atc aga ata gcc gat caa ttt     288
Glu Asp Met Arg Ile Asn Ala Glu His Ile Arg Ile Ala Asp Gln Phe
                85                  90                  95

gtg gaa gta cct ggt ggg acc aat aac aat aac tac gcc aat gtg cag     336
Val Glu Val Pro Gly Gly Thr Asn Asn Asn Asn Tyr Ala Asn Val Gln
            100                 105                 110

ctt att cta gag att gct gag ata act cac gtt gat gcg gtg tgg cct     384
Leu Ile Leu Glu Ile Ala Glu Ile Thr His Val Asp Ala Val Trp Pro
        115                 120                 125

ggt tgg ggt cat gca tca gaa aat cct gag ctt cca gat gca tta aaa     432
Gly Trp Gly His Ala Ser Glu Asn Pro Glu Leu Pro Asp Ala Leu Lys
    130                 135                 140

gca aag gga att gta ttc ctt gga cct cct gct ata tct atg gca gca     480
Ala Lys Gly Ile Val Phe Leu Gly Pro Pro Ala Ile Ser Met Ala Ala
145                 150                 155                 160

ttg gga gac aaa att ggt tcc tcg ttg att gct cag gca gca gaa gtt     528
Leu Gly Asp Lys Ile Gly Ser Ser Leu Ile Ala Gln Ala Ala Glu Val
                165                 170                 175

cca acc ctt cca tgg agt ggt tct cat gtg aaa att cct cca gaa agt     576
Pro Thr Leu Pro Trp Ser Gly Ser His Val Lys Ile Pro Pro Glu Ser
            180                 185                 190

gac ttg att act att cct gat gaa att tac cgt gca gca tgt gtt tat     624
Asp Leu Ile Thr Ile Pro Asp Glu Ile Tyr Arg Ala Ala Cys Val Tyr
        195                 200                 205

aca aca gaa gaa gca att gca agt tgt caa gta gta ggt tac cct gca     672
Thr Thr Glu Glu Ala Ile Ala Ser Cys Gln Val Val Gly Tyr Pro Ala
    210                 215                 220

atg att aag gca tct tgg ggt ggt ggc ggc aaa ggc ata aga aag gtt     720
Met Ile Lys Ala Ser Trp Gly Gly Gly Gly Lys Gly Ile Arg Lys Val
225                 230                 235                 240

cat aat gat gat gag gtt agg gca ttg ttc aag caa gtt caa ggt gaa     768
His Asn Asp Asp Glu Val Arg Ala Leu Phe Lys Gln Val Gln Gly Glu
```

```
                    245                 250                 255

gta cca ggc tca cct ata ttt ata atg aaa gtt gct tcc cag agc cga      816
Val Pro Gly Ser Pro Ile Phe Ile Met Lys Val Ala Ser Gln Ser Arg
                260                 265                 270

cat ctt gaa gtc caa ttg att tgc gat cag cac gga aat ttt gca gca      864
His Leu Glu Val Gln Leu Ile Cys Asp Gln His Gly Asn Phe Ala Ala
            275                 280                 285

ttg cac agc cgt gat tgt agt gtt caa aga agg cat caa aag att att      912
Leu His Ser Arg Asp Cys Ser Val Gln Arg Arg His Gln Lys Ile Ile
        290                 295                 300

gaa gag ggt ccc att act gta gca cct cca gaa acg gtg aaa gaa ctt      960
Glu Glu Gly Pro Ile Thr Val Ala Pro Pro Glu Thr Val Lys Glu Leu
305                 310                 315                 320

gaa cag gcg gct aga aga tta gct aaa tct gta aat tat gtg ggg gca     1008
Glu Gln Ala Ala Arg Arg Leu Ala Lys Ser Val Asn Tyr Val Gly Ala
                    325                 330                 335

gct acc gtt gag tat ctt tat agc atg gaa act ggc gag tac tac ttt     1056
Ala Thr Val Glu Tyr Leu Tyr Ser Met Glu Thr Gly Glu Tyr Tyr Phe
                340                 345                 350

tta gag ttg aac ccc cga cta cag gtt gag cat cct gtt act gaa tgg     1104
Leu Glu Leu Asn Pro Arg Leu Gln Val Glu His Pro Val Thr Glu Trp
            355                 360                 365

ata gct gag ata aat ctg cca gca gca caa gtt gca gtt ggg atg ggc     1152
Ile Ala Glu Ile Asn Leu Pro Ala Ala Gln Val Ala Val Gly Met Gly
        370                 375                 380

atc cca ctc tgg caa att cct gag att agg cgt ttc tat ggg atg gaa     1200
Ile Pro Leu Trp Gln Ile Pro Glu Ile Arg Arg Phe Tyr Gly Met Glu
385                 390                 395                 400

cat ggt ggg gga aat gat ggt tgg aag aaa aca tca gtg tta gct acc     1248
His Gly Gly Gly Asn Asp Gly Trp Lys Lys Thr Ser Val Leu Ala Thr
                    405                 410                 415

cct ttt gat ttt gac gaa gca caa tct aca aag ccg aaa ggt cat tgt     1296
Pro Phe Asp Phe Asp Glu Ala Gln Ser Thr Lys Pro Lys Gly His Cys
                420                 425                 430

gtg gct gta cga gtc acc agt gag gac ccc gat gat ggt ttt acg cct     1344
Val Ala Val Arg Val Thr Ser Glu Asp Pro Asp Asp Gly Phe Thr Pro
            435                 440                 445

aca gga gga aaa gtg cag gag ctc agc ttt aaa agc aag cca aat gtg     1392
Thr Gly Gly Lys Val Gln Glu Leu Ser Phe Lys Ser Lys Pro Asn Val
        450                 455                 460

tgg gct tat ttc tct gtt aag tcc gga gga gga att cat gaa ttc tca     1440
Trp Ala Tyr Phe Ser Val Lys Ser Gly Gly Gly Ile His Glu Phe Ser
465                 470                 475                 480

gat tct caa ttt gga cat gtt ttt gcg ttt gga gaa tct aga gct tta     1488
Asp Ser Gln Phe Gly His Val Phe Ala Phe Gly Glu Ser Arg Ala Leu
                    485                 490                 495
```

gca att gca aat atg gta ctg ggg ttg aag gaa att caa att cga gga 1536
Ala Ile Ala Asn Met Val Leu Gly Leu Lys Glu Ile Gln Ile Arg Gly
500 505 510

gaa att cgt acc aac gtt gat tac aca att gat ctt ctg aat gct tca 1584
Glu Ile Arg Thr Asn Val Asp Tyr Thr Ile Asp Leu Leu Asn Ala Ser
515 520 525

gac tac aga gac aac aaa att cac aca gga tgg cta gac agt aga att 1632
Asp Tyr Arg Asp Asn Lys Ile His Thr Gly Trp Leu Asp Ser Arg Ile
530 535 540

gca atg cgg gtt aga gca gag agg cct ccc tgg tat ctg tct gtt gtt 1680
Ala Met Arg Val Arg Ala Glu Arg Pro Pro Trp Tyr Leu Ser Val Val
545 550 555 560

ggt ggg gca ctc tat aaa gct tct gcc agc agt gca gct tta gtt tcg 1728
Gly Gly Ala Leu Tyr Lys Ala Ser Ala Ser Ser Ala Ala Leu Val Ser
565 570 575

gac tat gtt ggc tat ctt gaa aag ggg caa atc cct ccc aag cac att 1776
Asp Tyr Val Gly Tyr Leu Glu Lys Gly Gln Ile Pro Pro Lys His Ile
580 585 590

tct ctt gtc cat tct caa gtt tct ttg agc att gaa gga agc aaa tac 1824
Ser Leu Val His Ser Gln Val Ser Leu Ser Ile Glu Gly Ser Lys Tyr
595 600 605

acg att gac atg gta cga gga gga cct gga agt tac aaa ttg aaa ttg 1872
Thr Ile Asp Met Val Arg Gly Gly Pro Gly Ser Tyr Lys Leu Lys Leu
610 615 620

aat caa tcg gag ata gaa gcg gag ata cac act tta cgt gat gga ggt 1920
Asn Gln Ser Glu Ile Glu Ala Glu Ile His Thr Leu Arg Asp Gly Gly
625 630 635 640

ttg cta atg cag ttg gat gga aac agt cat gta ata tat gca gag gaa 1968
Leu Leu Met Gln Leu Asp Gly Asn Ser His Val Ile Tyr Ala Glu Glu
645 650 655

gaa gca gct gga act cgg ctt tta ata gat gga agg act tgc ttg ctt 2016
Glu Ala Ala Gly Thr Arg Leu Leu Ile Asp Gly Arg Thr Cys Leu Leu
660 665 670

cag aat gat gac gat cca tca aag tta att gga gag aca ccg tgc aag 2064
Gln Asn Asp Asp Asp Pro Ser Lys Leu Ile Gly Glu Thr Pro Cys Lys
675 680 685

ctt ctg aga tat ttg gtt gcg gat gat agt cag att gat gca gac aca 2112
Leu Leu Arg Tyr Leu Val Ala Asp Asp Ser Gln Ile Asp Ala Asp Thr
690 695 700

cca tat gct gaa gtt gag gtc atg aag atg tgc atg cct ctt ctt tcc 2160
Pro Tyr Ala Glu Val Glu Val Met Lys Met Cys Met Pro Leu Leu Ser
705 710 715 720

cct gct tct gga att att cat ttc aga atg gct gaa ggt caa gcc atg 2208
Pro Ala Ser Gly Ile Ile His Phe Arg Met Ala Glu Gly Gln Ala Met
725 730 735

```
cag gct ggt gaa ctt ata gca aag ctt gat cta gat gat ggt tct gca      2256
Gln Ala Gly Glu Leu Ile Ala Lys Leu Asp Leu Asp Asp Gly Ser Ala
                740                 745                 750

gta agg aag gca gaa ccc ttc act ggg agc ttc cct atc ctg ggc cct      2304
Val Arg Lys Ala Glu Pro Phe Thr Gly Ser Phe Pro Ile Leu Gly Pro
            755                 760                 765

cct act gca att tca ggt aaa gtt cat cag aaa tgt gca gca agc tta      2352
Pro Thr Ala Ile Ser Gly Lys Val His Gln Lys Cys Ala Ala Ser Leu
    770                 775                 780

aac gct gca cgg atg att ctt gct ggc tat gag cac aac att gat gaa      2400
Asn Ala Ala Arg Met Ile Leu Ala Gly Tyr Glu His Asn Ile Asp Glu
785                 790                 795                 800

gtt gtg gtc aaa agt ttg ctc aat tgc ctt gac agc cct gaa ctg cct      2448
Val Val Val Lys Ser Leu Leu Asn Cys Leu Asp Ser Pro Glu Leu Pro
                805                 810                 815

ttc ctt caa tgg caa gag tgc ttt gca gtt ttg gca acc cgt ctt ccc      2496
Phe Leu Gln Trp Gln Glu Cys Phe Ala Val Leu Ala Thr Arg Leu Pro
            820                 825                 830

aaa gat ctt aga aac gag ttg gaa gct aaa tat aag gag ttc gaa att      2544
Lys Asp Leu Arg Asn Glu Leu Glu Ala Lys Tyr Lys Glu Phe Glu Ile
        835                 840                 845

att tca agc tcc caa act att gat ttc cct gcc aaa tta ttg aag gca      2592
Ile Ser Ser Ser Gln Thr Ile Asp Phe Pro Ala Lys Leu Leu Lys Ala
    850                 855                 860

atc ctt gaa gct cat ctt tcc tcc tgt cct gaa aac gaa aaa gga gcc      2640
Ile Leu Glu Ala His Leu Ser Ser Cys Pro Glu Asn Glu Lys Gly Ala
865                 870                 875                 880

tta gaa aga cta gtt gaa ccg ctg aca agt ctt gta aag tct tat gag      2688
Leu Glu Arg Leu Val Glu Pro Leu Thr Ser Leu Val Lys Ser Tyr Glu
                885                 890                 895

ggt gga aga gag agc cat gct cat aaa att gtt caa tct cta ttt gaa      2736
Gly Gly Arg Glu Ser His Ala His Lys Ile Val Gln Ser Leu Phe Glu
            900                 905                 910

gag tat ctt tca gtt gaa gaa cta ttc agt gat aat ata cag gct gat      2784
Glu Tyr Leu Ser Val Glu Glu Leu Phe Ser Asp Asn Ile Gln Ala Asp
        915                 920                 925

gta att gaa cga ctc cgt ctt caa tac aag aaa gat ttg ttg aag att      2832
Val Ile Glu Arg Leu Arg Leu Gln Tyr Lys Lys Asp Leu Leu Lys Ile
    930                 935                 940

gta gat att gtg ctc tct cat cag ggt gtc aag agc aaa aac aag ctg      2880
Val Asp Ile Val Leu Ser His Gln Gly Val Lys Ser Lys Asn Lys Leu
945                 950                 955                 960

ata ctg cga cta atg gat aaa ctg gtt tac cct aat cct gct gcc tat      2928
Ile Leu Arg Leu Met Asp Lys Leu Val Tyr Pro Asn Pro Ala Ala Tyr
                965                 970                 975

agg gat caa tta atc cga ttc tcc caa ctc aac cat ata gtt tat tct      2976
```

```
Arg Asp Gln Leu Ile Arg Phe Ser Gln Leu Asn His Ile Val Tyr Ser
                980                 985                 990

gag ttg gct ctt aag gca agt caa ctg ttg gag caa act aaa ctc agt      3024
Glu Leu Ala Leu Lys Ala Ser Gln Leu Leu Glu Gln Thr Lys Leu Ser
            995                 1000                1005

gaa ctt cga tcc agc att gct aga agt ctt tct gaa cta gaa atg ttt      3072
Glu Leu Arg Ser Ser Ile Ala Arg Ser Leu Ser Glu Leu Glu Met Phe
    1010                1015                1020

acc gag gat ggt gaa aat att gat act ccg aag agg aag agt gcc att      3120
Thr Glu Asp Gly Glu Asn Ile Asp Thr Pro Lys Arg Lys Ser Ala Ile
1025                1030                1035                1040

aat gac aga atg gag gac ctt gtg agc gct cct ttg gct gtt gaa gat      3168
Asn Asp Arg Met Glu Asp Leu Val Ser Ala Pro Leu Ala Val Glu Asp
                1045                1050                1055

gcc ctt gtt ggt tta ttt gat cac agc gat cac acc ctt caa agg aga      3216
Ala Leu Val Gly Leu Phe Asp His Ser Asp His Thr Leu Gln Arg Arg
            1060                1065                1070

gtt gtt gaa act tat atc cgt agg ctc tat cag cca tat ctt gtc aaa      3264
Val Val Glu Thr Tyr Ile Arg Arg Leu Tyr Gln Pro Tyr Leu Val Lys
        1075                1080                1085

gat agc atc agg atg cag tgg cac aga tct ggc ctt att gct aca tgg      3312
Asp Ser Ile Arg Met Gln Trp His Arg Ser Gly Leu Ile Ala Thr Trp
    1090                1095                1100

gaa ttc tta gaa gaa tac gtt gaa cgg aag aat ggg gtt gaa gac aaa      3360
Glu Phe Leu Glu Glu Tyr Val Glu Arg Lys Asn Gly Val Glu Asp Lys
1105                1110                1115                1120

aca ctg gtg gag aaa cat agt gag aag aaa tgg gga gtg atg gtt gta      3408
Thr Leu Val Glu Lys His Ser Glu Lys Lys Trp Gly Val Met Val Val
                1125                1130                1135

att aaa tct ctt cag ttt ttg cca gca att atc agt gct gca tta aga      3456
Ile Lys Ser Leu Gln Phe Leu Pro Ala Ile Ile Ser Ala Ala Leu Arg
            1140                1145                1150

gaa gca acc aat aac ttt cac gat cct ctt aaa agt ggt tct ggt gac      3504
Glu Ala Thr Asn Asn Phe His Asp Pro Leu Lys Ser Gly Ser Gly Asp
        1155                1160                1165

tca agt aac cat ggt aat atg atg cat att gga tta gtg ggg atc aac      3552
Ser Ser Asn His Gly Asn Met Met His Ile Gly Leu Val Gly Ile Asn
    1170                1175                1180

aac caa atg agt tta ctt caa gac agt ggt gat gag gat cag gct caa      3600
Asn Gln Met Ser Leu Leu Gln Asp Ser Gly Asp Glu Asp Gln Ala Gln
1185                1190                1195                1200

gaa aga att gat aag ttg gcc aaa ata ctc aga gag cag gaa ata ggg      3648
Glu Arg Ile Asp Lys Leu Ala Lys Ile Leu Arg Glu Gln Glu Ile Gly
                1205                1210                1215

tcc ata ata cat gct gca ggt gtt gga gat att agc tgt atc ata cag      3696
Ser Ile Ile His Ala Ala Gly Val Gly Asp Ile Ser Cys Ile Ile Gln
```

```
            1220                1225                1230

agg gat gaa ggg cgt gct cca atg agg cat tcc ttt cac tgg tca tct     3744
Arg Asp Glu Gly Arg Ala Pro Met Arg His Ser Phe His Trp Ser Ser
        1235                1240                1245

gaa aag cta tat tat gta gag gaa cca ttg ttg ctc cat ctt gaa cct     3792
Glu Lys Leu Tyr Tyr Val Glu Glu Pro Leu Leu Leu His Leu Glu Pro
    1250                1255                1260

ccc cta tcc att tat ctt gaa ctg gac aag ctt aag tgc tat gaa aat     3840
Pro Leu Ser Ile Tyr Leu Glu Leu Asp Lys Leu Lys Cys Tyr Glu Asn
1265                1270                1275                1280

att cgc tat aca cca tcc cga gat cgt caa tgg cac ctc tac aca gtt     3888
Ile Arg Tyr Thr Pro Ser Arg Asp Arg Gln Trp His Leu Tyr Thr Val
                1285                1290                1295

gtg gat acc aag cca caa cca att caa aga atg ttt ctt cga aca ctt     3936
Val Asp Thr Lys Pro Gln Pro Ile Gln Arg Met Phe Leu Arg Thr Leu
            1300                1305                1310

atc aga cag cca acc aca aat gaa gga tac tct tct tat caa aga ctg     3984
Ile Arg Gln Pro Thr Thr Asn Glu Gly Tyr Ser Ser Tyr Gln Arg Leu
        1315                1320                1325

gat gca gaa acg tcc cgt acc caa ttg gct atg tct tat act tca agg     4032
Asp Ala Glu Thr Ser Arg Thr Gln Leu Ala Met Ser Tyr Thr Ser Arg
    1330                1335                1340

agc att ttt agg tcc cta atg ggc gca atg gag gag ttg gaa ctt aac     4080
Ser Ile Phe Arg Ser Leu Met Gly Ala Met Glu Glu Leu Glu Leu Asn
1345                1350                1355                1360

tca cac aat acc acc atc aaa tct gaa cat gct cat atg tac ctc tat     4128
Ser His Asn Thr Thr Ile Lys Ser Glu His Ala His Met Tyr Leu Tyr
                1365                1370                1375

atc ata cgc gag cag caa ata gat gat ctt gtg cct tat tcc aag aaa     4176
Ile Ile Arg Glu Gln Gln Ile Asp Asp Leu Val Pro Tyr Ser Lys Lys
            1380                1385                1390

att aac ata gaa gct ggc caa gaa gaa aca aca gtt gag gca atc ttg     4224
Ile Asn Ile Glu Ala Gly Gln Glu Glu Thr Thr Val Glu Ala Ile Leu
        1395                1400                1405

gaa gaa ctg gca cag gaa atc cat tcc tct gtt ggt gta aga atg cac     4272
Glu Glu Leu Ala Gln Glu Ile His Ser Ser Val Gly Val Arg Met His
    1410                1415                1420

aga tta ggc gtt ttc gtg tgg gaa atc aag ctc tgg att aca gca tgt     4320
Arg Leu Gly Val Phe Val Trp Glu Ile Lys Leu Trp Ile Thr Ala Cys
1425                1430                1435                1440

gga cag gca aat ggt gct tgg agg gtc att gta aac aat gtg act ggt     4368
Gly Gln Ala Asn Gly Ala Trp Arg Val Ile Val Asn Asn Val Thr Gly
                1445                1450                1455

cat aca tgc act gta cat ata tat cga gag atg gag gat gcc acc act     4416
His Thr Cys Thr Val His Ile Tyr Arg Glu Met Glu Asp Ala Thr Thr
            1460                1465                1470
```

```
cat aaa gtg gtc tac agt tca gtc act gta aag ggt ccg ttg cat ggt      4464
His Lys Val Val Tyr Ser Ser Val Thr Val Lys Gly Pro Leu His Gly
        1475                1480                1485

gta ccg gtg aat gaa aac tat caa cct ttg gga ggt att gac cga aaa      4512
Val Pro Val Asn Glu Asn Tyr Gln Pro Leu Gly Gly Ile Asp Arg Lys
    1490                1495                1500

cgt ctt gca gcg aga aag aac agc acc aca tac tgc tat gat ttc ccc      4560
Arg Leu Ala Ala Arg Lys Asn Ser Thr Thr Tyr Cys Tyr Asp Phe Pro
1505                1510                1515                1520

ctt gca ttt caa aca tcc ttg gaa cag tcc tgg tca ata cag cag aca      4608
Leu Ala Phe Gln Thr Ser Leu Glu Gln Ser Trp Ser Ile Gln Gln Thr
                1525                1530                1535

gga att caa aga gct aat gat aag gat ctc cta aaa gta aca gag ctt      4656
Gly Ile Gln Arg Ala Asn Asp Lys Asp Leu Leu Lys Val Thr Glu Leu
            1540                1545                1550

aaa ttt tcc gaa aaa gct ggt agt tgg ggt act tct ctt gtt cct gca      4704
Lys Phe Ser Glu Lys Ala Gly Ser Trp Gly Thr Ser Leu Val Pro Ala
        1555                1560                1565

gag cgt ctt cct gga ctc aat gat gtt ggc atg gta gcc tgg ttg atg      4752
Glu Arg Leu Pro Gly Leu Asn Asp Val Gly Met Val Ala Trp Leu Met
    1570                1575                1580

gaa atg tgt acg cct aaa ttc cca tct gga agg aca ata ttg gtt gtt      4800
Glu Met Cys Thr Pro Lys Phe Pro Ser Gly Arg Thr Ile Leu Val Val
1585                1590                1595                1600

tca aac gat gtg acc ttc aag gcc ggg tct ttt ggc cca aga gag gat      4848
Ser Asn Asp Val Thr Phe Lys Ala Gly Ser Phe Gly Pro Arg Glu Asp
                1605                1610                1615

gca ttc ttt aga gca gta act gat ctt gcc tgt gca aag aaa ata cct      4896
Ala Phe Phe Arg Ala Val Thr Asp Leu Ala Cys Ala Lys Lys Ile Pro
            1620                1625                1630

tta att tac ttg gca gca aat tct ggt gcc cgt tta ggt gtt gcc gag      4944
Leu Ile Tyr Leu Ala Ala Asn Ser Gly Ala Arg Leu Gly Val Ala Glu
        1635                1640                1645

gaa gtc aaa gct tgt ttc aaa gtt ggt tgg tct gag gaa tct aaa cct      4992
Glu Val Lys Ala Cys Phe Lys Val Gly Trp Ser Glu Glu Ser Lys Pro
    1650                1655                1660

gaa cat ggt ttt cag tat gta tat tta aca cct gag gat tat gct cga      5040
Glu His Gly Phe Gln Tyr Val Tyr Leu Thr Pro Glu Asp Tyr Ala Arg
1665                1670                1675                1680

atc gga tca tca gtg atg gca cat gaa tta aag ctt gaa agt gga gaa      5088
Ile Gly Ser Ser Val Met Ala His Glu Leu Lys Leu Glu Ser Gly Glu
                1685                1690                1695

acc aga tgg gtt ata gat acc att gtt ggc aaa gaa gat gga ctg gga      5136
Thr Arg Trp Val Ile Asp Thr Ile Val Gly Lys Glu Asp Gly Leu Gly
            1700                1705                1710
```

```
gtt gaa aac ttg agt ggt agt ggg gcc att gcc ggt gcc tat tca agg      5184
Val Glu Asn Leu Ser Gly Ser Gly Ala Ile Ala Gly Ala Tyr Ser Arg
        1715                1720                1725

gca tac aag gaa acc ttt aca ttg aca tat gtt acc ggt agg act gtt      5232
Ala Tyr Lys Glu Thr Phe Thr Leu Thr Tyr Val Thr Gly Arg Thr Val
    1730                1735                1740

gga att ggt gct tat ctt gct agg ctt ggg atg agg tgc ata cag agg      5280
Gly Ile Gly Ala Tyr Leu Ala Arg Leu Gly Met Arg Cys Ile Gln Arg
1745                1750                1755                1760

ctt gat caa cct ata att ctt acc ggg ttt tca gca tta aac aaa ctt      5328
Leu Asp Gln Pro Ile Ile Leu Thr Gly Phe Ser Ala Leu Asn Lys Leu
                1765                1770                1775

ctt ggt agg gag gtg tac agc tct cac atg caa ctt ggt gga ccg aaa      5376
Leu Gly Arg Glu Val Tyr Ser Ser His Met Gln Leu Gly Gly Pro Lys
            1780                1785                1790

atc atg gca aca aat gga gtc gtt cat ctc aca gtt tcg gac gac ctt      5424
Ile Met Ala Thr Asn Gly Val Val His Leu Thr Val Ser Asp Asp Leu
        1795                1800                1805

gaa ggc gtt tct tct att ttg aag tgg ctt agc tac gtt cct tct cat      5472
Glu Gly Val Ser Ser Ile Leu Lys Trp Leu Ser Tyr Val Pro Ser His
    1810                1815                1820

gta ggt ggt gca ctt ccc att gta aag ccc ctt gat ccc cca gag agg      5520
Val Gly Gly Ala Leu Pro Ile Val Lys Pro Leu Asp Pro Pro Glu Arg
1825                1830                1835                1840

gaa gtg gag tat tta ccg gaa aat tca tgc gat cct cgt gct gcc att      5568
Glu Val Glu Tyr Leu Pro Glu Asn Ser Cys Asp Pro Arg Ala Ala Ile
                1845                1850                1855

tcc gga act ctg gat gtt aat gga aag tgg ctg gga ggc att ttt gac      5616
Ser Gly Thr Leu Asp Val Asn Gly Lys Trp Leu Gly Gly Ile Phe Asp
            1860                1865                1870

aag gac agc ttt gtg gag aca cta gaa gga tgg gct aga aca gtt gtt      5664
Lys Asp Ser Phe Val Glu Thr Leu Glu Gly Trp Ala Arg Thr Val Val
        1875                1880                1885

aca gga agg gca aag ctt gga gga atc cct gtg gga att gtt gcg gtg      5712
Thr Gly Arg Ala Lys Leu Gly Gly Ile Pro Val Gly Ile Val Ala Val
    1890                1895                1900

gaa aca caa aca gtt atg caa ata ata cct gct gat cca ggt caa ctt      5760
Glu Thr Gln Thr Val Met Gln Ile Ile Pro Ala Asp Pro Gly Gln Leu
1905                1910                1915                1920

gat tct cac gag agg gtt gtt cct caa gcc ggg cag gtg tgg ttt cct      5808
Asp Ser His Glu Arg Val Val Pro Gln Ala Gly Gln Val Trp Phe Pro
                1925                1930                1935

gat tct gcg acc aag acg gcc caa gcg ata ttg gat ttc aac aga gaa      5856
Asp Ser Ala Thr Lys Thr Ala Gln Ala Ile Leu Asp Phe Asn Arg Glu
            1940                1945                1950

gaa ctc cca ctt ttc att atc gca aac tgg aga ggc ttt tca ggt gga      5904
```

Glu Leu Pro Leu Phe Ile Ile Ala Asn Trp Arg Gly Phe Ser Gly Gly
1955 1960 1965

caa agg gac ctt ttt gaa gga att ctt cag gct ggt tcg act att gtg 5952
Gln Arg Asp Leu Phe Glu Gly Ile Leu Gln Ala Gly Ser Thr Ile Val
1970 1975 1980

gag aac ctt agg aca tac aaa cag ccc ata ttt gta tac att cca atg 6000
Glu Asn Leu Arg Thr Tyr Lys Gln Pro Ile Phe Val Tyr Ile Pro Met
1985 1990 1995 2000

atg ggt gaa ctc cga ggc ggg gct tgg gtt gtt gtc gac agc cga atc 6048
Met Gly Glu Leu Arg Gly Gly Ala Trp Val Val Val Asp Ser Arg Ile
2005 2010 2015

aac tca gac cac att gaa atg tat gct gag cga acg gcc aaa ggt aac 6096
Asn Ser Asp His Ile Glu Met Tyr Ala Glu Arg Thr Ala Lys Gly Asn
2020 2025 2030

gtc ctt gag ccg gaa gga atg att gaa atc aaa ttt aga aca aga gaa 6144
Val Leu Glu Pro Glu Gly Met Ile Glu Ile Lys Phe Arg Thr Arg Glu
2035 2040 2045

ttg ttg gag tgt atg aga aga ctt gat caa caa ttg att aat ttg aag 6192
Leu Leu Glu Cys Met Arg Arg Leu Asp Gln Gln Leu Ile Asn Leu Lys
2050 2055 2060

gaa aaa ctt tct gaa gcc aag agt aac aag gac tat ggt gca tat gat 6240
Glu Lys Leu Ser Glu Ala Lys Ser Asn Lys Asp Tyr Gly Ala Tyr Asp
2065 2070 2075 2080

tct ctg cag cag cag att aga ttc cgt gag aaa cag ctt ttg cct ttg 6288
Ser Leu Gln Gln Gln Ile Arg Phe Arg Glu Lys Gln Leu Leu Pro Leu
2085 2090 2095

tat act cag ata gct aca aaa ttt gct gaa ctc cat gat act tca tta 6336
Tyr Thr Gln Ile Ala Thr Lys Phe Ala Glu Leu His Asp Thr Ser Leu
2100 2105 2110

aga atg aaa gca aag ggt gta atc aga gaa gtt ctt gat tgg cgt aag 6384
Arg Met Lys Ala Lys Gly Val Ile Arg Glu Val Leu Asp Trp Arg Lys
2115 2120 2125

tcg cgt tct gtc ttc tat cag aga ctg cac agg aga atc ggt gag cac 6432
Ser Arg Ser Val Phe Tyr Gln Arg Leu His Arg Arg Ile Gly Glu His
2130 2135 2140

tca ctg atc aac atc gtg aga gat gct gct ggt gac caa ttg tca tat 6480
Ser Leu Ile Asn Ile Val Arg Asp Ala Ala Gly Asp Gln Leu Ser Tyr
2145 2150 2155 2160

gtt tct gcc atg aac ttg ctc aaa gaa tgg tat ctg aat tct gat atc 6528
Val Ser Ala Met Asn Leu Leu Lys Glu Trp Tyr Leu Asn Ser Asp Ile
2165 2170 2175

gcc aaa ggt aga gaa gat gct tgg ttg gac gat gaa gcc ttc ttc aga 6576
Ala Lys Gly Arg Glu Asp Ala Trp Leu Asp Asp Glu Ala Phe Phe Arg
2180 2185 2190

tgg agg gat gat cca gca aac tac gag gat aaa cta aag gaa ttg cgc 6624
Trp Arg Asp Asp Pro Ala Asn Tyr Glu Asp Lys Leu Lys Glu Leu Arg

```
        2195                2200                2205

gtc cag aga ctg ttg ctt cag ttg aca aat att ggc gac tcg gct cta      6672
Val Gln Arg Leu Leu Leu Gln Leu Thr Asn Ile Gly Asp Ser Ala Leu
    2210                2215                2220

gat tta caa gct cta cct caa ggt ctt gcc gcc ctt tta agc aag ttg      6720
Asp Leu Gln Ala Leu Pro Gln Gly Leu Ala Ala Leu Leu Ser Lys Leu
2225                2230                2235                2240

gaa gca tca agt cgc gat aag ttg atc agt gaa ctt cgc aaa gta ctc      6768
Glu Ala Ser Ser Arg Asp Lys Leu Ile Ser Glu Leu Arg Lys Val Leu
                2245                2250                2255

ggt tagtagacag tgaatgctcc tgtgatctgc ccatgcactc atgttgtagt           6821
Gly

gttcacgtcg ttgatacatg accatataga aatgtatcca ttttacgatg ttatcatcaa    6881
agtagcagca tccctcggaa aatggacttt cacttgaggg atcaactgta aatgacttcg    6941
gtcttggata gatatttaat ttatgcagtt agaggatcat aaccagcatc accatgtttg    7001
gtctatttat ttgctggttg attgattctt tgcgtgtatc tgaataaaca tgtaataatt    7061
tgtaacattg attatttttt atgaaaaaca aagttttggg cactcctttt ataaaaaaaa    7121
aaaaaagaat tcctgcagcc cgggggatcc                                     7151
```

<210> 8
<211> 2257
<212> PRT
<213> Medicago sativa

<400> 8

```
Met Ala Ser Val Gly Arg Gly Asn Gly Tyr Leu Asn Ser Val Leu Pro
 1               5                  10                  15
Ser Arg His Pro Ala Thr Thr Thr Glu Val Asp Glu Tyr Cys Asn Ala
            20                  25                  30
Leu Gly Gly Asn Lys Pro Ile His Ser Ile Leu Ile Ala Asn Asn Gly
        35                  40                  45
Met Ala Ala Val Lys Phe Ile Arg Ser Val Arg Ser Trp Ala Tyr Glu
    50                  55                  60
Thr Phe Gly Thr Glu Lys Ala Ile Leu Leu Val Ala Met Ala Thr Pro
65                  70                  75                  80
Glu Asp Met Arg Ile Asn Ala Glu His Ile Arg Ile Ala Asp Gln Phe
                85                  90                  95
Val Glu Val Pro Gly Gly Thr Asn Asn Asn Asn Tyr Ala Asn Val Gln
            100                 105                 110
Leu Ile Leu Glu Ile Ala Glu Ile Thr His Val Asp Ala Val Trp Pro
        115                 120                 125
Gly Trp Gly His Ala Ser Glu Asn Pro Glu Leu Pro Asp Ala Leu Lys
    130                 135                 140
Ala Lys Gly Ile Val Phe Leu Gly Pro Pro Ala Ile Ser Met Ala Ala
145                 150                 155                 160
Leu Gly Asp Lys Ile Gly Ser Ser Leu Ile Ala Gln Ala Ala Glu Val
                165                 170                 175
Pro Thr Leu Pro Trp Ser Gly Ser His Val Lys Ile Pro Pro Glu Ser
            180                 185                 190
Asp Leu Ile Thr Ile Pro Asp Glu Ile Tyr Arg Ala Ala Cys Val Tyr
        195                 200                 205
Thr Thr Glu Glu Ala Ile Ala Ser Cys Gln Val Val Gly Tyr Pro Ala
    210                 215                 220
Met Ile Lys Ala Ser Trp Gly Gly Gly Gly Lys Gly Ile Arg Lys Val
225                 230                 235                 240
```

His Asn Asp Asp Glu Val Arg Ala Leu Phe Lys Gln Val Gln Gly Glu
245 250 255
Val Pro Gly Ser Pro Ile Phe Ile Met Lys Val Ala Ser Gln Ser Arg
260 265 270
His Leu Glu Val Gln Leu Ile Cys Asp Gln His Gly Asn Phe Ala Ala
275 280 285
Leu His Ser Arg Asp Cys Ser Val Gln Arg Arg His Gln Lys Ile Ile
290 295 300
Glu Glu Gly Pro Ile Thr Val Ala Pro Pro Glu Thr Val Lys Glu Leu
305 310 315 320
Glu Gln Ala Ala Arg Arg Leu Ala Lys Ser Val Asn Tyr Val Gly Ala
325 330 335
Ala Thr Val Glu Tyr Leu Tyr Ser Met Glu Thr Gly Glu Tyr Tyr Phe
340 345 350
Leu Glu Leu Asn Pro Arg Leu Gln Val Glu His Pro Val Thr Glu Trp
355 360 365
Ile Ala Glu Ile Asn Leu Pro Ala Ala Gln Val Ala Val Gly Met Gly
370 375 380
Ile Pro Leu Trp Gln Ile Pro Glu Ile Arg Arg Phe Tyr Gly Met Glu
385 390 395 400
His Gly Gly Gly Asn Asp Gly Trp Lys Lys Thr Ser Val Leu Ala Thr
405 410 415
Pro Phe Asp Phe Asp Glu Ala Gln Ser Thr Lys Pro Lys Gly His Cys
420 425 430
Val Ala Val Arg Val Thr Ser Glu Asp Pro Asp Asp Gly Phe Thr Pro
435 440 445
Thr Gly Gly Lys Val Gln Glu Leu Ser Phe Lys Ser Lys Pro Asn Val
450 455 460
Trp Ala Tyr Phe Ser Val Lys Ser Gly Gly Gly Ile His Glu Phe Ser
465 470 475 480
Asp Ser Gln Phe Gly His Val Phe Ala Phe Gly Glu Ser Arg Ala Leu
485 490 495
Ala Ile Ala Asn Met Val Leu Gly Leu Lys Glu Ile Gln Ile Arg Gly
500 505 510
Glu Ile Arg Thr Asn Val Asp Tyr Thr Ile Asp Leu Leu Asn Ala Ser
515 520 525
Asp Tyr Arg Asp Asn Lys Ile His Thr Gly Trp Leu Asp Ser Arg Ile
530 535 540
Ala Met Arg Val Arg Ala Glu Arg Pro Pro Trp Tyr Leu Ser Val Val
545 550 555 560
Gly Gly Ala Leu Tyr Lys Ala Ser Ala Ser Ser Ala Ala Leu Val Ser
565 570 575
Asp Tyr Val Gly Tyr Leu Glu Lys Gly Gln Ile Pro Pro Lys His Ile
580 585 590
Ser Leu Val His Ser Gln Val Ser Leu Ser Ile Glu Gly Ser Lys Tyr
595 600 605
Thr Ile Asp Met Val Arg Gly Gly Pro Gly Ser Tyr Lys Leu Lys Leu
610 615 620
Asn Gln Ser Glu Ile Glu Ala Glu Ile His Thr Leu Arg Asp Gly Gly
625 630 635 640
Leu Leu Met Gln Leu Asp Gly Asn Ser His Val Ile Tyr Ala Glu Glu
645 650 655
Glu Ala Ala Gly Thr Arg Leu Leu Ile Asp Gly Arg Thr Cys Leu Leu
660 665 670
Gln Asn Asp Asp Asp Pro Ser Lys Leu Ile Gly Glu Thr Pro Cys Lys
675 680 685
Leu Leu Arg Tyr Leu Val Ala Asp Asp Ser Gln Ile Asp Ala Asp Thr
690 695 700
Pro Tyr Ala Glu Val Glu Val Met Lys Met Cys Met Pro Leu Leu Ser
705 710 715 720
Pro Ala Ser Gly Ile Ile His Phe Arg Met Ala Glu Gly Gln Ala Met

725 730 735
Gln Ala Gly Glu Leu Ile Ala Lys Leu Asp Leu Asp Asp Gly Ser Ala
740 745 750
Val Arg Lys Ala Glu Pro Phe Thr Gly Ser Phe Pro Ile Leu Gly Pro
755 760 765
Pro Thr Ala Ile Ser Gly Lys Val His Gln Lys Cys Ala Ala Ser Leu
770 775 780
Asn Ala Ala Arg Met Ile Leu Ala Gly Tyr Glu His Asn Ile Asp Glu
785 790 795 800
Val Val Val Lys Ser Leu Leu Asn Cys Leu Asp Ser Pro Glu Leu Pro
805 810 815
Phe Leu Gln Trp Gln Glu Cys Phe Ala Val Leu Ala Thr Arg Leu Pro
820 825 830
Lys Asp Leu Arg Asn Glu Leu Glu Ala Lys Tyr Lys Glu Phe Glu Ile
835 840 845
Ile Ser Ser Ser Gln Thr Ile Asp Phe Pro Ala Lys Leu Leu Lys Ala
850 855 860
Ile Leu Glu Ala His Leu Ser Ser Cys Pro Glu Asn Glu Lys Gly Ala
865 870 875 880
Leu Glu Arg Leu Val Glu Pro Leu Thr Ser Leu Val Lys Ser Tyr Glu
885 890 895
Gly Gly Arg Glu Ser His Ala His Lys Ile Val Gln Ser Leu Phe Glu
900 905 910
Glu Tyr Leu Ser Val Glu Glu Leu Phe Ser Asp Asn Ile Gln Ala Asp
915 920 925
Val Ile Glu Arg Leu Arg Leu Gln Tyr Lys Lys Asp Leu Leu Lys Ile
930 935 940
Val Asp Ile Val Leu Ser His Gln Gly Val Lys Ser Lys Asn Lys Leu
945 950 955 960
Ile Leu Arg Leu Met Asp Lys Leu Val Tyr Pro Asn Pro Ala Ala Tyr
965 970 975
Arg Asp Gln Leu Ile Arg Phe Ser Gln Leu Asn His Ile Val Tyr Ser
980 985 990
Glu Leu Ala Leu Lys Ala Ser Gln Leu Leu Glu Gln Thr Lys Leu Ser
995 1000 1005
Glu Leu Arg Ser Ser Ile Ala Arg Ser Leu Ser Glu Leu Glu Met Phe
1010 1015 1020
Thr Glu Asp Gly Glu Asn Ile Asp Thr Pro Lys Arg Lys Ser Ala Ile
1025 1030 1035 1040
Asn Asp Arg Met Glu Asp Leu Val Ser Ala Pro Leu Ala Val Glu Asp
1045 1050 1055
Ala Leu Val Gly Leu Phe Asp His Ser Asp His Thr Leu Gln Arg Arg
1060 1065 1070
Val Val Glu Thr Tyr Ile Arg Arg Leu Tyr Gln Pro Tyr Leu Val Lys
1075 1080 1085
Asp Ser Ile Arg Met Gln Trp His Arg Ser Gly Leu Ile Ala Thr Trp
1090 1095 1100
Glu Phe Leu Glu Glu Tyr Val Glu Arg Lys Asn Gly Val Glu Asp Lys
1105 1110 1115 1120
Thr Leu Val Glu Lys His Ser Glu Lys Lys Trp Gly Val Met Val Val
1125 1130 1135
Ile Lys Ser Leu Gln Phe Leu Pro Ala Ile Ile Ser Ala Ala Leu Arg
1140 1145 1150
Glu Ala Thr Asn Asn Phe His Asp Pro Leu Lys Ser Gly Ser Gly Asp
1155 1160 1165
Ser Ser Asn His Gly Asn Met Met His Ile Gly Leu Val Gly Ile Asn
1170 1175 1180
Asn Gln Met Ser Leu Leu Gln Asp Ser Gly Asp Glu Asp Gln Ala Gln
1185 1190 1195 1200
Glu Arg Ile Asp Lys Leu Ala Lys Ile Leu Arg Glu Gln Glu Ile Gly
1205 1210 1215

Ser Ile Ile His Ala Ala Gly Val Gly Asp Ile Ser Cys Ile Ile Gln
1220 1225 1230
Arg Asp Glu Gly Arg Ala Pro Met Arg His Ser Phe His Trp Ser Ser
1235 1240 1245
Glu Lys Leu Tyr Tyr Val Glu Glu Pro Leu Leu Leu His Leu Glu Pro
1250 1255 1260
Pro Leu Ser Ile Tyr Leu Glu Leu Asp Lys Leu Lys Cys Tyr Glu Asn
1265 1270 1275 1280
Ile Arg Tyr Thr Pro Ser Arg Asp Arg Gln Trp His Leu Tyr Thr Val
1285 1290 1295
Val Asp Thr Lys Pro Gln Pro Ile Gln Arg Met Phe Leu Arg Thr Leu
1300 1305 1310
Ile Arg Gln Pro Thr Thr Asn Glu Gly Tyr Ser Ser Tyr Gln Arg Leu
1315 1320 1325
Asp Ala Glu Thr Ser Arg Thr Gln Leu Ala Met Ser Tyr Thr Ser Arg
1330 1335 1340
Ser Ile Phe Arg Ser Leu Met Gly Ala Met Glu Glu Leu Glu Leu Asn
1345 1350 1355 1360
Ser His Asn Thr Thr Ile Lys Ser Glu His Ala His Met Tyr Leu Tyr
1365 1370 1375
Ile Ile Arg Glu Gln Gln Ile Asp Asp Leu Val Pro Tyr Ser Lys Lys
1380 1385 1390
Ile Asn Ile Glu Ala Gly Gln Glu Glu Thr Thr Val Glu Ala Ile Leu
1395 1400 1405
Glu Glu Leu Ala Gln Glu Ile His Ser Ser Val Gly Val Arg Met His
1410 1415 1420
Arg Leu Gly Val Phe Val Trp Glu Ile Lys Leu Trp Ile Thr Ala Cys
1425 1430 1435 1440
Gly Gln Ala Asn Gly Ala Trp Arg Val Ile Val Asn Asn Val Thr Gly
1445 1450 1455
His Thr Cys Thr Val His Ile Tyr Arg Glu Met Glu Asp Ala Thr Thr
1460 1465 1470
His Lys Val Val Tyr Ser Ser Val Thr Val Lys Gly Pro Leu His Gly
1475 1480 1485
Val Pro Val Asn Glu Asn Tyr Gln Pro Leu Gly Gly Ile Asp Arg Lys
1490 1495 1500
Arg Leu Ala Ala Arg Lys Asn Ser Thr Thr Tyr Cys Tyr Asp Phe Pro
1505 1510 1515 1520
Leu Ala Phe Gln Thr Ser Leu Glu Gln Ser Trp Ser Ile Gln Gln Thr
1525 1530 1535
Gly Ile Gln Arg Ala Asn Asp Lys Asp Leu Leu Lys Val Thr Glu Leu
1540 1545 1550
Lys Phe Ser Glu Lys Ala Gly Ser Trp Gly Thr Ser Leu Val Pro Ala
1555 1560 1565
Glu Arg Leu Pro Gly Leu Asn Asp Val Gly Met Val Ala Trp Leu Met
1570 1575 1580
Glu Met Cys Thr Pro Lys Phe Pro Ser Gly Arg Thr Ile Leu Val Val
1585 1590 1595 1600
Ser Asn Asp Val Thr Phe Lys Ala Gly Ser Phe Gly Pro Arg Glu Asp
1605 1610 1615
Ala Phe Phe Arg Ala Val Thr Asp Leu Ala Cys Ala Lys Lys Ile Pro
1620 1625 1630
Leu Ile Tyr Leu Ala Ala Asn Ser Gly Ala Arg Leu Gly Val Ala Glu
1635 1640 1645
Glu Val Lys Ala Cys Phe Lys Val Gly Trp Ser Glu Glu Ser Lys Pro
1650 1655 1660
Glu His Gly Phe Gln Tyr Val Tyr Leu Thr Pro Glu Asp Tyr Ala Arg
1665 1670 1675 1680
Ile Gly Ser Ser Val Met Ala His Glu Leu Lys Leu Glu Ser Gly Glu
1685 1690 1695
Thr Arg Trp Val Ile Asp Thr Ile Val Gly Lys Glu Asp Gly Leu Gly

```
            1700                1705                1710
Val Glu Asn Leu Ser Gly Ser Gly Ala Ile Ala Gly Ala Tyr Ser Arg
        1715                1720                1725
Ala Tyr Lys Glu Thr Phe Thr Leu Thr Tyr Val Thr Gly Arg Thr Val
    1730                1735                1740
Gly Ile Gly Ala Tyr Leu Ala Arg Leu Gly Met Arg Cys Ile Gln Arg
1745                1750                1755                1760
Leu Asp Gln Pro Ile Ile Leu Thr Gly Phe Ser Ala Leu Asn Lys Leu
                1765                1770                1775
Leu Gly Arg Glu Val Tyr Ser Ser His Met Gln Leu Gly Gly Pro Lys
            1780                1785                1790
Ile Met Ala Thr Asn Gly Val Val His Leu Thr Val Ser Asp Asp Leu
        1795                1800                1805
Glu Gly Val Ser Ser Ile Leu Lys Trp Leu Ser Tyr Val Pro Ser His
    1810                1815                1820
Val Gly Gly Ala Leu Pro Ile Val Lys Pro Leu Asp Pro Pro Glu Arg
1825                1830                1835                1840
Glu Val Glu Tyr Leu Pro Glu Asn Ser Cys Asp Pro Arg Ala Ala Ile
                1845                1850                1855
Ser Gly Thr Leu Asp Val Asn Gly Lys Trp Leu Gly Gly Ile Phe Asp
            1860                1865                1870
Lys Asp Ser Phe Val Glu Thr Leu Glu Gly Trp Ala Arg Thr Val Val
        1875                1880                1885
Thr Gly Arg Ala Lys Leu Gly Gly Ile Pro Val Gly Ile Val Ala Val
    1890                1895                1900
Glu Thr Gln Thr Val Met Gln Ile Ile Pro Ala Asp Pro Gly Gln Leu
1905                1910                1915                1920
Asp Ser His Glu Arg Val Val Pro Gln Ala Gly Gln Val Trp Phe Pro
                1925                1930                1935
Asp Ser Ala Thr Lys Thr Ala Gln Ala Ile Leu Asp Phe Asn Arg Glu
            1940                1945                1950
Glu Leu Pro Leu Phe Ile Ile Ala Asn Trp Arg Gly Phe Ser Gly Gly
        1955                1960                1965
Gln Arg Asp Leu Phe Glu Gly Ile Leu Gln Ala Gly Ser Thr Ile Val
    1970                1975                1980
Glu Asn Leu Arg Thr Tyr Lys Gln Pro Ile Phe Val Tyr Ile Pro Met
1985                1990                1995                2000
Met Gly Glu Leu Arg Gly Gly Ala Trp Val Val Val Asp Ser Arg Ile
                2005                2010                2015
Asn Ser Asp His Ile Glu Met Tyr Ala Glu Arg Thr Ala Lys Gly Asn
            2020                2025                2030
Val Leu Glu Pro Glu Gly Met Ile Glu Ile Lys Phe Arg Thr Arg Glu
        2035                2040                2045
Leu Leu Glu Cys Met Arg Arg Leu Asp Gln Gln Leu Ile Asn Leu Lys
    2050                2055                2060
Glu Lys Leu Ser Glu Ala Lys Ser Asn Lys Asp Tyr Gly Ala Tyr Asp
2065                2070                2075                2080
Ser Leu Gln Gln Gln Ile Arg Phe Arg Glu Lys Gln Leu Leu Pro Leu
                2085                2090                2095
Tyr Thr Gln Ile Ala Thr Lys Phe Ala Glu Leu His Asp Thr Ser Leu
            2100                2105                2110
Arg Met Lys Ala Lys Gly Val Ile Arg Glu Val Leu Asp Trp Arg Lys
        2115                2120                2125
Ser Arg Ser Val Phe Tyr Gln Arg Leu His Arg Arg Ile Gly Glu His
    2130                2135                2140
Ser Leu Ile Asn Ile Val Arg Asp Ala Ala Gly Asp Gln Leu Ser Tyr
2145                2150                2155                2160
Val Ser Ala Met Asn Leu Leu Lys Glu Trp Tyr Leu Asn Ser Asp Ile
                2165                2170                2175
Ala Lys Gly Arg Glu Asp Ala Trp Leu Asp Asp Glu Ala Phe Phe Arg
            2180                2185                2190
```

Trp Arg Asp Asp Pro Ala Asn Tyr Glu Asp Lys Leu Lys Glu Leu Arg
2195 2200 2205
Val Gln Arg Leu Leu Leu Gln Leu Thr Asn Ile Gly Asp Ser Ala Leu
2210 2215 2220
Asp Leu Gln Ala Leu Pro Gln Gly Leu Ala Ala Leu Leu Ser Lys Leu
2225 2230 2235 2240
Glu Ala Ser Ser Arg Asp Lys Leu Ile Ser Glu Leu Arg Lys Val Leu
2245 2250 2255
Gly

<210> 9
<211> 20
<212> DNA
<213> Artificial Sequence

<220>
<223> primer for PCR

<400> 9
gtaggcaccc tgctactaca 20

<210> 10
<211> 23
<212> DNA
<213> Artificial Sequence

<220>
<223> primer for PCR

<400> 10
catcaggaat agtaatcaag tca 23

<210> 11
<211> 46
<212> DNA
<213> Artificial Sequence

<220>
<223> representative construct (3' end)

<400> 11
ccttttataa aaaaaaaaaa aagaattcct gcagcccggg ggatcc 46

<210> 12
<211> 46
<212> DNA
<213> Artificial Sequence

<220>
<223> representative construct (3' end)

<400> 12
ccttttataa aaaaaaaaaa aagaattcct gcagcccggg ggatcc 46